# FASCICULE DE BREVET EUROPEEN

(11) **EP 3 808 425 B1**
(45) Date de publication et mention de la délivrance du brevet: **13.08.2025**
(21) Numéro de dépôt: 20207624.6
(22) Date de dépôt: 18.01.2016
(51) Int. Cl.: B01D 11/00, C02F 1/68, G21C 19/46, C02F 1/10, C02F 1/38, C02F 103/08

(54) **MÉTHODE DE DÉSSALEMENT D'EAU PAR DÉIONISATION THERMIQUE ET LIQUIDE D'EXTRACTION IONIQUE EN PHASE LIQUIDE**
VERFAHREN ZUM ENTSALZEN VON WASSER MITTELS THERMISCHER DEIONISIERUNG UND FLÜSSIGPHASENIONENEXTRAKTIONSFLÜSSIGKEIT
METHOD FOR THE DESALINATION OF WATER BY MEANS OF THERMAL DEIONISATION AND LIQUID-PHASE ION EXTRACTION LIQUID

(30) Priorité: 19.01.2015 FR 1550391
(43) Date de publication de la demande: 21.04.2021
(62) Demande divisionnaire de: 16703343.0
(73) Titulaire: ADIONICS, 94320 Thiais (FR)
(72) Inventeur: DE SOUZA, Guillaume, 91800 Brunoy (FR); POUESSEL, Jacky, 91470 Forges les Bains (FR); DAUTRICHE, Bastien, 49000 Angers (FR); CHTCHIGROVSKY, Mélanie, 91190 Gif-sur-yvette (FR); MEIRIES, Sébastien, 83210 Belgentier (FR)
(74) Mandataire: Cabinet Chaillot

(56) Documents cités:
- CH-A5- 609 558
- US-A- 3 331 874
- US-A- 3 407 056
- US-A- 3 408 290
- US-A1- 2008 014 133
- US-A1- 2008 179 568
- US-B1- 6 322 702
- US-B1- 6 566 561
- TATIANA G. LEVITSKAIA ET AL: "Synergistic Pseudo-Hydroxide Extraction: Synergism and Anion Selectivity in Sodium Extraction Using a Crown Ether and a Series of Weak Lipophilic Acids", ANALYTICAL CHEMISTRY, vol. 75, no. 3, 1 February 2003 (2003-02-01), pages 405 - 412, XP055210681, ISSN: 0003-2700, DOI: 10.1021/ac0259212

## Description

### Domaine technique de l'invention

Le domaine technique de l'invention est l'extraction ionique appliquée au dessalement d'eau, notamment de l'eau de mer.

### Art antérieur

L'approche courante de dessalement de l'eau de mer comprend l'extraction de l'eau de l'eau salée. Elle comprend les technologies d'évaporation/condensation de l'eau par chauffage naturel ou forcé, à pression ambiante ou sous vide et l'utilisation de membranes semi-perméables (Nanofiltration, Osmose inverse...). Quelle que soit la technologie, cette approche cumule les inconvénients et insuffisances suivantes :
**1.** Un niveau de valorisation de l'eau limité du fait qu'au-delà de 53% d'extraction d'eau d'une eau de mer courante, il y a entartrage des équipements par précipitation de CaCO₃, puis de CaSO₄, voire de Mg(OH)₂ et autres sels de faibles solubilités relatives contenues dans l'eau résiduelle. Si la technologie employée est associée à une vaporisation thermique de cette eau, la température d'usage, généralement dépassant les 80°C est alors génératrice d'une baisse du seuil de précipitation de certains sels (par exemple du CaCO₃ par évaporation du dioxyde de carbone) et des sels à solubilité inversée (CaSO₄ dans l'eau), ce qui limite d'autant plus le niveau maximum d'extraction de l'eau de l'eau salée pour n'atteindre alors que 30-35%.
2. Un mode opératoire couteux en énergie. L'eau étant largement majoritaire (en %masse et en %mole) par rapport aux ions dissous, extraire l'eau de l'eau salée revient à déplacer une grande quantité de matière ce qui n'est thermodynamiquement pas du tout favorable. Ainsi, la vaporisation de l'eau est extrêmement coûteuse en énergie (sa chaleur latente de vaporisation est de 2319 kJ/kg à 75°C. Ce qui est équivalent à bruler 74,6 mL d'essence par litre d'eau vaporisée alors qu'une eau de mer standard ne contient que 36 g de sels par kg d'eau de mer. Ainsi, afin de réduire l'énergie thermique consommée, il a été développé des technologies à effets multiples ou à détentes successives employant des cuves sous vide pour réduire l'énergie thermique consommée d'un ordre de grandeur et arriver ainsi à descendre à 230 kJ/kg avec 12 effets associés à un surplus d'investissement. De même, pour la perméation membranaire ou Osmose inverse (représentant plus de 90% des nouvelles capacités installées en 2011) l'énergie électrique consommée est comprise entre 3,5 et 4,5 kWh/m³ d'eau de mer dessalée.
3. L'utilisation des aciers inoxydables, usage nécessaire au vu des pressions opératoires qui sont soit très inférieures à la pression atmosphérique (sous vide) soit très supérieures (jusqu'à 80 bars) et de la forte concentration en chlorures. Ces produits sont coûteux, mais peuvent quand même se corroder et relarguer leurs composants métalliques, présents en surface, générant une pollution des eaux par des métaux toxiques de type chrome, nickel, molybdène, manganèse et cuivre.

Une autre approche du dessalement de l'eau consiste à extraire le sel de l'eau salée. Cette approche est employée dans le dessalement d'eau de faible salinité (< 3-5 g/L) en utilisant des membranes d'électrodialyse, ou pour obtenir de l'eau ultra pure à partir d'eau potable par emploi de résines échangeuses d'ions. Cette approche est également développée pour une technologie plus récente, en développement, basée sur le principe d'une déionisation capacitive (CDI ou CapDI), applicable aujourd'hui au seul dessalement d'eaux de faible salinité, dites saumâtres. La limitation technologique et économique de ces systèmes tient essentiellement au transfert et/ou au stockage d'ions au niveau des membranes, des résines ou des électrodes. Une surface de stockage très élevée combinée à des temps de cycles trop longs, ne permet pas aujourd'hui à ces technologies de déionisation capacitive d'être déployées en traitement d'eau de mer et sont donc limitées aux faibles salinités.

Les procédés d'extraction liquide-liquide, aussi appelés procédés d'extraction par solvant sont aujourd'hui industriellement employés en tant que technique de séparation en génie chimique. Pour la séparation de composés ioniques, il est aujourd'hui courant de séparer des composés organiques acides ou basiques, ou de purifier des métaux (Zn, Ni, Cu, Co, Cr, Mn...) après leur dissolution (lixiviation) dans l'eau (Hydrométallurgie). Cette technique de séparation est aussi employée pour l'obtention de produits de haute pureté comme des sels d'uranium, de plutonium, de césium, de strontium ou des sels de terres rares via un procédé d'échange liquide-liquide de cations.

Une bibliographie très abondante existe dans ce domaine dans laquelle on peut citer l'article de T. G. Levitskaia, -et al. Anal. Chem. 2003, 75, 405-412 qui démontre qu'il est possible d'extraire de la soude (NaOH) d'une solution aqueuse par un emploi d'un extractant neutre du sodium, de type éther-couronne, avec un acide faible lipophile dé-protonable pour permettre la formation d'un alcoolate de sodium hydrophobe.

[DC18C6]_{(org)} + [RCOH]_{(org)} + [Na⁺]_{(aq)} + [OH⁻]_{(aq)} ↔ [RCO⁻Na⁺DC18C6]_{(org)} + H₂O_{(aq)}

Ce document présente également des exemples d'extraction de NaF, NaCl, NaBr, NaNO₃ et de NaClO₄, à 1 M de salinité, par combinaison de DC18C6 à 0,02M sans, puis avec sept acides faibles (de la famille des alcools), présents à hauteur de 0,04M, le tout dissous dans du nitrobenzène. Deux de ces alcools sont des alcools aromatiques fluorés dont le pKa est d'environs 8,8. Le taux d'extraction pour des ions hydrophobes tels que le picrate, est relativement élevé. Cependant pour des anions hydrophiles, tel l'ion chlorure Cl⁻, les taux d'extractions recalculés sont compris entre 0,06% et 0,16%, ce qui confirme la grande difficulté d'extraire le NaCl hydrophile de l'eau et le peu d'influence des alcools, à cette concentration, sur la performance d'extraction.

Il a déjà été proposé dans la demande WO 2010/086575, l'utilisation dans un échangeur à contact direct comprenant une phase fluorée liquide et hydrophobe associée à des échangeurs d'ions, tels que des échangeurs d'ions, fluorés. Cependant la phase fluorée organique liquide décrite dans cette demande décrit l'emploi de composés organo-fluorés ioniques peu adaptés à l'obtention de taux de dessalement d'eau élevés, par exemple plus de 50%, de préférence plus de 70 % pour des sels alcalins hydrophiles tels que le NaCl à 0,2M à 25°C avec un faible coût opératoire et une faible demande énergétique.

L'invention a pour but de remédier à ces inconvénients en fournissant une nouvelle génération de phases liquides hydrophobes ayant une capacité d'absorption en espèces ioniques suffisamment dépendante de la température pour permettre, une extraction à basse température (par exemple à température ambiante) et une désextraction à chaud ces deux étapes présentant un différentiel de température, ΔT, supérieur à 30°C, de préférence de 50°C. D'autres aspects de l'invention portent sur des procédés et dispositifs de traitement de l'eau permettant la purification de l'eau à faible bilan énergétique.

### Description de l'invention

Ainsi, la présente invention concerne un procédé de traitement d'une eau saline par déionisation thermique comprenant l'extraction d'au moins deux espèces ioniques, lesdites espèces ioniques comprenant une espèce anionique et une espèce cationique et étant présentes dans de l'eau à traiter, ledit procédé comprenant les étapes suivantes :
a) le mélange dans un premier réacteur, à une première température, entre une phase organique hydrophobe liquide et l'eau saline à traiter, ladite eau à traiter étant à l'état liquide, pour l'obtention subséquente d'une eau traitée liquide dessalée et/ou déionisée en tout ou partie et d'une phase organique liquide hydrophobe chargée en lesdites espèces ioniques, ladite phase hydrophobe comprenant :
   - au moins un premier composé organique solvatant les anions, protique et hydrophobe dont le pKa dans l'eau à 25°C est d'au moins 9, de préférence 10,5 et est préférentiellement inférieur au pKa de l'eau à 25°C, ou du moins inférieur à 15 à 25°C et dont la solubilité dans l'eau à 25°C est inférieure à 0,01 mol/l, ledit premier composé étant un composé représenté par la Formule (B) : 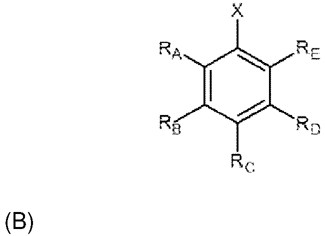
      dans laquelle au moins un quelconque des radicaux R_{A}, R_{B}, R_{C}, R_{D} et R_{E} identiques ou différents, est un atome halogène ou un groupement électro-attracteur du groupe suivant : F, CI, Br ; CₘF₂ₘ₊₁ avec m ≤ 4, où m est un entier non nul ; CF₂CF₂CₚH₂ₚ₊₁ avec p ≤ 4, où p est un entier ; CF₂CₚH₂ₚ₊₁ avec p ≤ 4, où p est un entier ; CH₂CₚF₂ₚ₊₁ avec p ≤ 4, où p est un entier ; OCH₂CF₃ ; C(=O)CF₃ ; CₘHₙFₚCl_{q}Brₛ avec m ≤ 4, où n, p, q, s sont des entiers dont au moins p, q ou s est non nul ; C(=O)OCₘH₂ₘ₊₁ avec m ≤ 4, où m est un entier ; et C(=O)CₘH₂ₘ₊₁ avec m ≤ 4, où m est un entier,
      le ou les radicaux R_{A}, R_{B}, R_{C}, R_{D} et R_{E} restants sont choisis, identiques ou différents, parmi les radicaux non électro-attracteurs suivants : H ; CH₃ ; CH₂CH₃ ; CH₂CH₂CₚF₂ₚ₊₁ avec p ≤ 4, ou p est un entier ; CₘH₂ₘ₋₁ avec m ≤ 10, où m est un entier non nul ; et CₘH₂ₘ₊₁ avec m ≤ 10, où m est un entier non nul ; où un seul des radicaux R_{A} à R_{E} peut être un de ces deux derniers radicaux CₘH₂ₘ₋₁ et CₘH₂ₘ₊₁ ;
      et dans laquelle X est choisi parmi les radicaux suivants : OH; 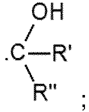 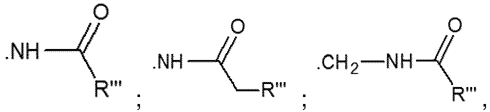
      où R' et R", identiques ou différents, sont choisis parmi les radicaux suivants : CₙH₂ₙ₋₁ avec n ≤ 4, où n est un entier non nul ; CₙH₂ₙ₊₁ avec n ≤ 4, où n est un entier non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; CₘHₙFₚCl_{q}Brₛ avec m ≤ 4, où n, p, q, s sont des entiers dont au moins p, q ou s est non nul ; et un radical aryle de formule (b) : 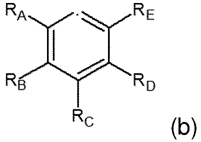
      où R_{A}, R_{B}, R_{C}, R_{D} et R_{E}, identiques ou différents, sont tels que précédemment définis dans la formule (B) ;
      et où R"' est choisi parmi les radicaux suivants : CₘH₂ₘ₊₁ avec m ≤ 20, où m est un entier ; CₘH₂ₘ₋₁ avec m ≤ 20, où m est un entier non nul ; CₘHₙFₚCl_{q}Brₛ avec m ≤ 10, où n, p, q, s sont des entiers dont au moins p, q ou s est non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; et un radical aryle de formule (b) : 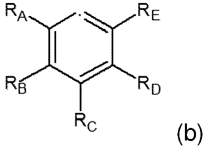
      où R_{A}, R_{B}, R_{C}, R_{D} et R_{E}, identiques ou différents, sont tels que précédemment définis dans la formule (B) ; et,
   - au moins un deuxième composé organique extractant les cations et hydrophobe choisi parmi les éther-couronnes, les cryptands ou les calixarènes fonctionnalisés et présentant une constante de complexation de ladite espèce cationique dont la valeur Log K, dans le méthanol à 25 °C, est supérieure à 3 et inférieure à 9 ;
b) la séparation, d'une part, de ladite eau traitée liquide dessalée et/ou déionisée en tout ou partie et d'autre part de ladite phase organique liquide chargée en lesdites espèces ioniques ; et
c) le mélange, à une deuxième température plus élevée, en phase liquide, dans un deuxième réacteur de ladite phase organique liquide chargée en lesdites espèces ioniques et d'eau de régénération liquide, pour l'obtention subséquente d'une phase organique liquide régénérée et d'eau liquide de régénération chargée en lesdites espèces ioniques, la différence entre lesdites première et deuxième températures allant de 30 °C à 150 °C, la deuxième température étant plus élevée que la première température.

Dans un mode de réalisation particulier, le procédé peut ne pas comprendre d'étape où le pH de l'eau de régénération liquide est significativement modifié, c'est-à-dire au-delà d'une variation de pH de +/- 2, par exemple +/- 1 par rapport à l'eau à traiter.

Le procédé selon l'invention peut également comprendre les étapes subséquentes de :
d) séparation de ladite phase organique liquide régénérée et de l'eau liquide de régénération chargée en lesdites espèces ioniques ;
e) mise en contact thermique indirecte de ladite phase organique liquide chargée en lesdites espèces ioniques et de ladite phase organique liquide régénérée.

Le procédé peut comprendre une étape de chauffage de l'eau de régénération liquide effectuée avant l'étape c).

L'espèce anionique peut être du chlorure ou du sulfate.

Le composé (B) peut être un composé dans lequel X représente :

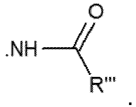

En particulier, le composé (B) peut être représenté par la formule : 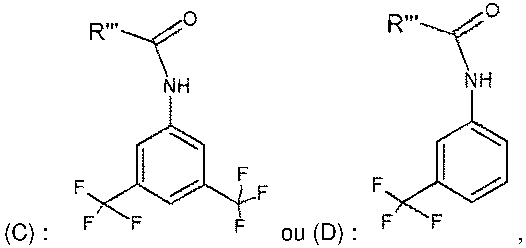
dans laquelle R"' est choisi parmi les radicaux suivants : CₘH₂ₘ₊₁ avec m ≤ 20, de préférence ≤ 15 où m est un entier ; CₘH₂ₘ₋₁ avec m ≤ 20, où m est un entier non nul ; CₘHₙFₚCl_{q}Brₛ avec m ≤ 10, où n, p, q, s sont des entiers dont au moins p, q ou s est non nul ; et un radical aryle de formule (b) : 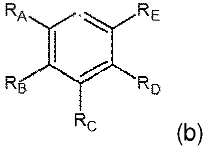
dans laquelle au moins un quelconque des radicaux R_{A}, R_{B}, R_{C}, R_{D} et R_{E}, identiques ou différents, est un atome halogène ou un groupement électro-attracteur, en particulier un radical halogéné, du groupe suivant : F, CI, Br ; CₘF₂ₘ₊₁ avec m ≤ 4, où m est un entier non nul ; CF₂CF₂CₚH₂ₚ₊₁ avec p ≤ 4, où p est un entier ; CF₂CₚH₂ₚ₊₁ avec p ≤ 4, où p est un entier ; CH₂CₚF₂ₚ₊₁ avec p ≤ 4, où p est un entier ; OCH₂CF₃ ; C(=O)CF₃ ; CₘHₙFₚCl_{q}Brₛ avec m ≤ 4, où n, p, q, s sont des entiers dont au moins p, q ou s est non nul ; C(=O)OCₘH₂ₘ₊₁ avec m ≤ 4, où m est un entier ; et C(=O)CₘH₂ₘ₊₁ avec m ≤ 4, où m est un entier, le ou les radicaux R_{A}, R_{B}, R_{C}, R_{D} et R_{E} restants sont choisis, identiques ou différents, parmi les radicaux non électro-attracteurs suivants : H ; CH₃ ; CH₂CH₃ ; CH₂CH₂CₚF_{2p + 1} avec p ≤ 4, ou p est un entier ; CₘH₂ₘ₋₁ avec m ≤ 10, où m est un entier non nul ; et CₘH₂ₘ₊₁ avec m ≤ 10, où m est un entier non nul,
où un seul des radicaux R_{A} à R_{E} peut être un de ces deux derniers radicaux CₘH₂ₘ₋₁ et CₘH_{2m+1.}

En particulier, le radical R"' peut être n-C₇H₁₅, n-C₉H₁₉, n-C₁₁H₂₃ ou n-C₁₃H₂₇.

Le deuxième composé organique peut être un éther couronne, notamment un éther couronne ayant 14 à 80 atomes de carbone, et peut être choisi dans le groupe constitué du 6,7,9,10,12,13,20,21,23,24-décahydrodibenzo[b,k][1,4,7,10,12,16,19] heptaoxa-cyclohenicosine (DB21C7), benzo[b]-1,4,7,10,13-pentaoxacyclopentadécane (B15C5), perhydrobenzo[b]-1,4,7,10,13-pentaoxacyclopentadécane (C15C5), dicyclohexano-1,4,7,10,13,16-hexaoxacyclooctadécane (DC18C6), dibenzo[b,k]-1,4,7,10,13,16-hexaoxacyclooctadécane (DB18C6) et 6,7,9,10,12,13,20,21,23,24,26,27-dodécahydrodibenzo[b,n][1,4,7,10,13,16,19,22]octaoxa-cyclotétracosine (DB24C8).

Le deuxième composé organique peut être un calixarène substitué pouvant comprendre, par exemple, de 32 à 80 atomes de carbone, par exemple de 50 à 70 atomes de carbone, tel que l'ester tétraéthylique de l'acide 4-tert-butylcalix[4]-arène-O',O',O",O"'-tétraacétique, tel que le Calix[4]Est.

La phase organique hydrophobe liquide utilisée à l'étape a) peut également contenir un fluidifiant.

Le fluidifiant peut être choisi dans le groupe constitué de solvants aromatiques polaires.

La présente invention porte égalemet sur une composition pour la mise en œuvre du procédé selon l'invention, caractérisé par le fait qu'elle comprend :
- au moins un premier composé organique solvatant les anions, protique et hydrophobe dont le pKa dans l'eau à 25°C est d'au moins 9, de préférence 10,5 et est préférentiellement inférieur au pKa de l'eau à 25°C, ou du moins inférieur à 15 à 25°C et dont la solubilité dans l'eau à 25°C est inférieure à 0,01 mol/l, ledit premier composé étant un composé représenté par la Formule (B) : 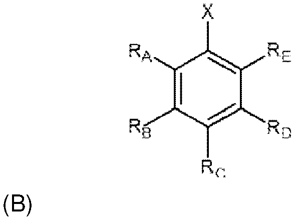
- dans laquelle au moins un quelconque des radicaux R_{A}, R_{B}, R_{C}, R_{D} et R_{E} identiques ou différents, est un atome halogène ou un groupement électro-attracteur du groupe suivant : F, CI, Br ; CₘF₂ₘ₊₁ avec m ≤ 4, où m est un entier non nul ; CF₂CF₂CₚH₂ₚ₊₁ avec p ≤ 4, où p est un entier ; CF₂CₚH₂ₚ₊₁ avec p ≤ 4, où p est un entier ; CH₂CₚF₂ₚ₊₁ avec p ≤ 4, où p est un entier ; OCH₂CF₃ ; C(=O)CF₃ ; CₘHₙFₚCl_{q}Brₛ avec m ≤ 4, où n, p, q, s sont des entiers dont au moins p, q ou s est non nul ; C(=O)OCₘH₂ₘ₊₁ avec m ≤ 4, où m est un entier ; et C(=O)CₘH₂ₘ₊₁ avec m ≤ 4, où m est un entier,
- le ou les radicaux R_{A}, R_{B}, R_{C}, R_{D} et R_{E} restants sont choisis, identiques ou différents, parmi les radicaux non électro-attracteurs suivants : H ; CH₃ ; CH₂CH₃ ; CH₂CH₂CₚF₂ₚ₊₁ avec p ≤ 4, ou p est un entier ; CₘH₂ₘ₋₁ avec m ≤ 10, où m est un entier non nul ; et CₘH₂ₘ₊₁ avec m ≤ 10, où m est un entier non nul ; où un seul des radicaux R_{A} à R_{E} peut être un de ces deux derniers radicaux CₘH₂ₘ₋₁ et CₘH₂ₘ₊₁ ;
- et dans laquelle X est choisi parmi les radicaux suivants : OH; 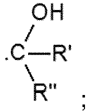 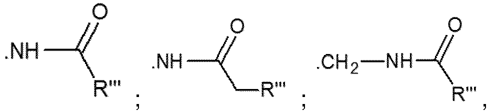
- où R' et R", identiques ou différents, sont choisis parmi les radicaux suivants : CₙH₂ₙ₋₁ avec n ≤ 4, où n est un entier non nul ; CₙH₂ₙ₊₁ avec n ≤ 4, où n est un entier non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; CₘHₙFₚCl_{q}Brₛ avec m ≤ 4, où n, p, q, s sont des entiers dont au moins p, q ou s est non nul ; et un radical aryle de formule (b) : 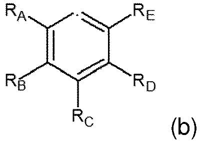
- où R_{A}, R_{B}, R_{C}, R_{D} et R_{E}, identiques ou différents, sont tels que précédemment définis dans la formule (B) ;
- et où R"' est choisi parmi les radicaux suivants : CₘH₂ₘ₊₁ avec m ≤ 20, où m est un entier ; CₘH₂ₘ₋₁ avec m ≤ 20, où m est un entier non nul ; CₘHₙFₚCl_{q}Brₛ avec m ≤ 10, où n, p, q, s sont des entiers dont au moins p, q ou s est non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; et un radical aryle de formule (b) : 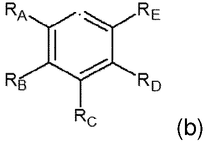
- où R_{A}, R_{B}, R_{C}, R_{D} et R_{E}, identiques ou différents, sont tels que précédemment définis dans la formule (B)
- au moins un second composé organique hydrophobe extractant les cations choisi parmi les éther-couronnes, les cryptands ou les calixarènes fonctionnalisés et présentant une constante de complexation de ladite espèce cationique dont la valeur Log K, dans le méthanol à 25 °C, est supérieure à 3 et inférieure à 9.

Le premier composé est un composé permettant de solvater une espèce anionique, qui est désigné par l'acronyme MSA (pour Molécule Solvatante d'Anion). Le deuxième composé est un composé permettant d'extraire (par exemple solvater ou chélater) une espèce cationique qui est désigné par l'acronyme MEC (pour Molécule Extractante de Cation). De manière surprenante l'association de MSA et MEC selon l'invention permet l'extraction (ou la solvatation) de cations et plus particulièrement d'anions hydrophiles particulièrement difficiles à transférer dans une phase organique.

Les termes « espèces anioniques » et « espèces cationiques » sont respectivement équivalents aux termes « anions » et « cations ».

Le pKa (ou constante d'acidité) est défini par pKa = -log₁₀ Ka, où Ka est la constante de dissociation acide qui est mesurée de manière standard pour de tels pKa. La méthode de mesure standard recommandée pour des pKa élevés, basiques, est de préférence celle décrite par Popov et al, IUPAC - Guidelines for NMR measurements for determination of high and low pKa Pure Appl.Chem., Vol. 78, No3, pp 663_675, 2006.

K est la constante de complexation d'une MEC et d'un cation dans le méthanol, à 25°C, qui est mesuré selon la méthode standard de titration calorimétrique isotherme.

Par le terme « hydrophobe » on entend un composé, ou un mélange de composés, dont la solubilité dans l'eau, à 25°C, est au moins inférieure à 0,1 Mol/Litre. De préférence il est choisi des composés hydrophobes dont la solubilité dans l'eau à 25°C est inférieure à 0,01 Mol/L, de préférence inférieure à 0,0001 Mol/L et avantageusement de moins de 1x10⁻⁵ Mol/L. L'hydrophobicité ou la solubilité d'un composé peut être mesuré par des méthodes standard et notamment par spectrométrie UV-visible.

Alternativement le pKa du premier composé est choisi dans une gamme allant de 12 à 15, de préférence 13 à 14. Par gamme de pKa allant de 12 à 15 on entend des pKa de 12,1; 12,2; 12,3; 12,4; 12,5; 12,6; 12,7; 12,8; 12,9; 13,0; 13,1; 13,2; 13,3; 13,4; 13,5; 13,6; 13,7; 13,8; 13,9; 14,0; 14,1; 14,2; 14,3; 14,4; 14,5; 14,6; 14,7; 14,8; 14,9 ou de 15,0.

Selon un aspect préférentiel, le second composé, permettant l'extraction, dans la composition, d'au moins un cation, présente une constante de complexation Log K pour ledit cation allant de 4 à 8, de préférence de 5 à 7. Par Log K allant de 5 à 7, on entend 5,1 ; 5,2 ; 5,3 ; 5,4 ; 5,5 ; 5,6 ; 5,7 ; 5,8 ; 5,9 ; 6,0; 6,1 ; 6,2 ; 6,3 ; 6,4 ; 6,5 ; 6,6 ; 6,7 ; 6,8 ; 6,9 ou de 7,0.

Avantageusement, ce second composé présente également une constante de complexation pour le sodium, dans l'eau à 25°C, supérieure ou égale à 1.

### Composé MSA

Le MSA est un composé de formule B : 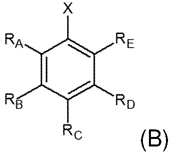
dans laquelle au moins un quelconque des radicaux R_{A}, R_{B}, R_{C}, R_{D} et R_{E}, identiques ou différents, est un atome halogène ou un groupement électro-attracteurs, en particulier un radical halogéné, du groupe suivant :
   - F, CI, Br,
   - CₘF₂ₘ₊₁ avec m ≤ 4, où m est un entier non nul,
   - CF₂CF₂CₚH₂ₚ₊₁ avec p ≤ 4, où p est un entier,
   - CF₂CₚH₂ₚ₊₁ avec p ≤ 4, où p est un entier,
   - CH₂CₚF₂ₚ₊₁ avec p ≤ 4, où p est un entier,
   - OCH₂CF₃,
   - C(=O)CF₃,
   - CₘHₙFₚCl_{q}Brₛ avec m ≤ 4, où n, p, q, s sont des entiers dont au moins p, q ou s est non nul,
   - C(=O)OCₘH₂ₘ₊₁ avec m ≤ 4, où m est un entier, et
   - C(=O)CₘH₂ₘ₊₁ avec m ≤ 4, où m est un entier,
le ou les radicaux R_{A}, R_{B}, R_{C}, R_{D} et R_{E} restant(s) sont choisis, identiques ou différents, parmi les radicaux non électro-attracteurs suivants :
   - H,
   - CH₃,
   - CH₂CH₃,
   - CH₂CH₂CₚF₂ₚ₊₁ avec p ≤ 4, où p est un entier,
   - CₘH₂ₘ₋₁ avec m ≤ 10, où m est un entier non nul, et
   - CₘH₂ₘ₊₁ avec m ≤ 10, où m est un entier non nul ; où un seul des radicaux R_{A} à R_{E} peut être un de ces deux derniers radicaux CₘH₂ₘ₋₁ et CₘH₂ₘ₊₁ ;
et dans lequel X est choisi parmi les radicaux suivants :
   - OH, 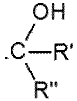 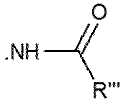 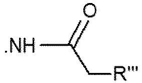 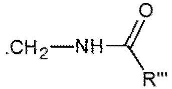 ou R' et R", identiques ou différents, sont choisis parmi les radicaux suivants :
   - CₙH₂ₙ₋₁ avec n ≤ 4, où n est un entier non nul,
   - CₙH₂ₙ₊₁ avec n ≤ 4, où n est un entier non nul,
   - CH₂CH₂CₚF₂ₚ₊₁ avec p ≤ 4, où p est un entier,
   - CH₂CₚF₂ₚ₊₁ avec p ≤ 4, où p est un entier,
   - CF₂CₚH₂ₚ₊₁ avec p ≤ 4, où p est un entier,
   - CF₂CF₂CₚH₂ₚ₊₁ avec p ≤ 4, où p est un entier,
   - CₘF₂ₘ₊₁ avec m ≤ 4, où m est un entier non nul,
   - CₘHₙFₚCl_{q}Brₛ avec m ≤ 4, où n, p, q, s sont des entiers dont au moins p, q ou s est non nul,
   - et un radical aryle de formule b : 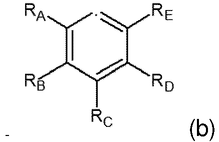 où R_{A}, R_{B}, R_{C}, R_{D} et R_{E}, identiques ou différents, sont tels que précédemment définis dans la formule B;
et dans laquelle R"' est choisi parmi les radicaux suivants :
   - CₘH₂ₘ₊₁ avec m ≤ 20, de préférence ≤ 15, où m est un entier,
   - CₘH₂ₘ₋₁ avec m ≤ 20, où m est un entier non nul,
   - CₘHₙFₚCl_{q}Brₛ avec m ≤ 10, où n, p, q, s sont des entiers dont au moins p, q ou s est non nul,
   - CH₂CH₂CₚF₂ₚ₊₁ avec p ≤ 4, où p est un entier,
   - CH₂CₚF₂ₚ₊₁ avec p ≤ 4, où p est un entier,
   - CF₂CₚH₂ₚ₊₁ avec p ≤ 4, où m est un entier,
   - CF₂CF₂CₚH₂ₚ₊₁ avec p ≤ 4, où m est un entier,
   - CₘF₂ₘ₊₁ avec m ≤ 4, où m est un entier non nul,
   - et un radical aryle de formule b : 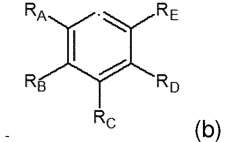 où R_{A}, R_{B}, R_{C}, R_{D} et R_{E}, identiques ou différents, sont tels que précédemment définis dans la formule B.

### Composé MSA - alcool

Un tel composé est avantageusement choisi dans le groupe des alcools aromatiques fluorés. Par exemple ce composé peut-être un dérivé du phénol, tel que le 3-(trifluorométhyl)phénol (N° CAS : 98-17-9).

De préférence, ce premier composé est un composé phényle méthanolique qui comprend avantageusement plus de 3 atomes de fluor. Avantageusement ce composé comprend plus de deux radicaux -CF₃.

Selon un mode de réalisation de l'invention ce premier composé est un composé de formule A : 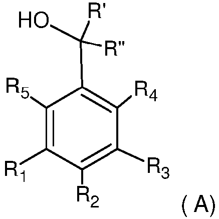 dans laquelle
R₁, R₂, R₃, R₄ et R₅, identiques ou différents, mais où l'un quelconque de R₁, R₂ et R₃ est un radical fluoré, sont choisis parmi les radicaux suivants :
   - H,
   - F,
   - CₘF₂ₘ₊₁ avec m ≤ 4, où m est un entier non nul,
   - CF₂CₚH₂ₚ₊₁ avec p ≤ 4, où p est un entier non nul, et
   - CF₂CF₂CₚH₂ₚ₊₁ avec p ≤ 4, où p est un entier non nul ;
et dans laquelle R' et R", identiques ou différents, sont choisis parmi les radicaux suivants :
   - CₙH₂ₙ₋₁ avec n ≤ 4, où n est un entier non nul,
   - CₙH₂ₙ₊₁ avec n ≤ 4, où n est un entier non nul,
   - CH₂CₚF₂ₚ₊₁ où p ≤ 2, où p est un entier non nul,
   - CH₂CH₂CₚF₂ₚ₊₁ où p ≤ 2, où p est un entier non nul,
   - et un radical aryle de formule a : 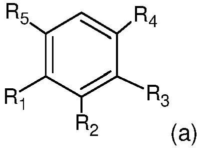 où R₁, R₂, R₃, R₄ et R₅, identiques ou différents, sont choisis dans le groupe
      - H
      - F
      - CₘF₂ₘ₊₁ avec m ≤ 4,.
      - CF₂CₚH₂ₚ₊₁ avec p ≤ 4, où p est un entier non nul,
      - CF₂CF₂CₚH₂ₚ₊₁ avec p ≤ 4, où p est un entier non nul.

Avantageusement ledit premier composé est choisi dans le groupe constitué par les composés décrits dans le tableau I suivant :

**Tableau I :**

| **MSA Formule semi développée** | **Formule brute n° CAS** | **Masse molaire (g/mole)** | **Densité (g/cm3)** | **[MSA] maximale Mole/L** | **Solubilité à l'eau mMole/L** | **pKa** |
|---|---|---|---|---|---|---|
| 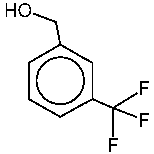 (Référence) | C8H7F3O | 176,14 | 1,29 | 7,32 | 32 | 14,6 +/- 1,0 (estimé) |
| | 349-75-7 | | Liquide | | | |
| 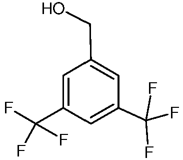 (Référence) | C9H6F6O | 244,13 | 1,43 | 5,86 | 2,29 | 14,5 +/- 1,0 (estimé) |
| | 32707-89-4 | | Solide | | | |
| 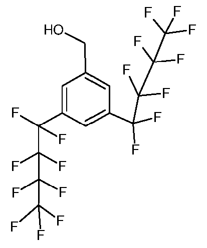 (Référence) | C15H6F18O | 544,18 | 1,62 | 2,98 | 0,0005 | 14,01 +/- 0,1 |
| | 916975-23-0 | | | | | |
| 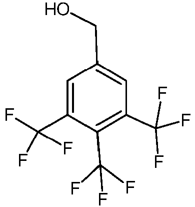 (Référence) | C10H5F9O | 312,13 | 1,53 | 4,90 | 0,39 | 13,59 +/- 0,1 |
| | 1010101-84-4 | | | | | |
| 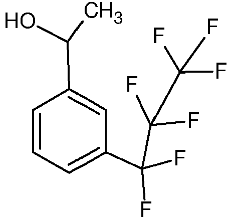 (Référence) | C11H9F7O | 290,18 | 1,39 | 4,70 | 0,42 | 14,5 +/- 1,0 (estimé) |
| | 131608-30-5 | | | | | |
| 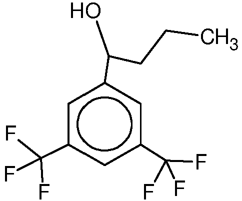 (Référence) | C12H12F6O | 286,21 | 1,30 | 4,54 | 0,48 | 13,9 +/- 1,0 (estimé) |
| | 742097-71-8 | | Liquide | | | |
| 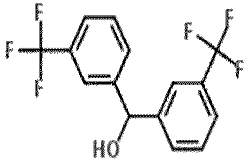 | C15H10F6O | 320,23 | 1,37 | 4,28 | 0,07 | 13,3 +/- 1,0 (estimé) |
| | 1598-89-6 | | Liquide | | | |

et les composés MSA 2, MSA 3 et MSA 5 décrits ci-après.

Selon un aspect de l'invention, la composition liquide organique hydrophobe comprend au moins deux composés permettant la solvatation d'au moins un anion. De préférence, ces composés sont choisis parmi les composés de type (MSA) décrits dans la présente demande.

### Composé MSA - amide

Le composé MSA de formule B peut également être un composé amide. Dans ce cas le radical X dans la formule B, est : 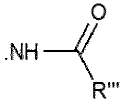 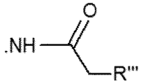 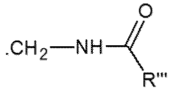 où R"' est tel que décrit précédemment.

De préférence l'amide est de formule : 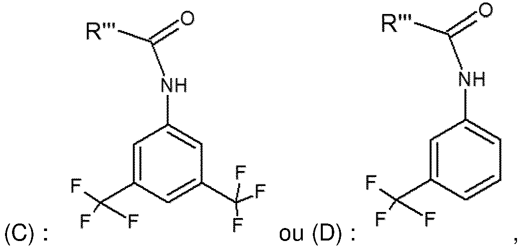 dans laquelle R‴ est choisi parmi les radicaux suivants :
- -CₘH₂ₘ₊₁ avec m ≤ 20, de préférence ≤ 15 où m est un entier,
- -CₘH₂ₘ₋₁ avec m ≤ 20, où m est un entier non nul,
- -CₘHₙFₚCl_{q}Brₛ avec m ≤ 10, où n, p, q, s sont des entiers dont au moins p, q ou s est non nul,
- et un radical aryle de formule b :

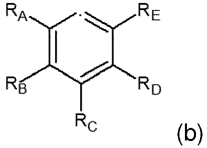

dans laquelle au moins un quelconque des radicaux R_{A}, R_{B}, R_{C}, R_{D} et R_{E}, identiques ou différents, est un atome halogène ou un groupement électro-attracteurs, en particulier un radical halogéné, du groupe suivant :
   - F, Cl, Br,
   - CₘF₂ₘ₊₁ avec m ≤ 4, où m est un entier non nul,
   - CF₂CF₂CₚH₂ₚ₊₁ avec p ≤ 4, où p est un entier,
   - CF₂CₚH₂ₚ₊₁ avec p ≤ 4, où p est un entier,
   - CH₂CₚF₂ₚ₊₁ avec p ≤ 4, où p est un entier,
   - OCH₂CF₃,
   - C(=O)CF₃,
   - CₘHₙFₚCl_{q}Brₛ avec m ≤ 4, où n, p, q, s sont des entiers dont au moins p, q ou s est non nul,
   - C(=O)OCₘH₂ₘ₊₁ avec m ≤ 4, où m est un entier, et
   - C(=O)CₘH₂ₘ₊₁ avec m ≤ 4, où m est un entier,
le ou les radicaux R_{A}, R_{B}, R_{C}, R_{D} et R_{E} restant(s) sont choisis, identiques ou différents, parmi les radicaux non électro-attracteurs suivants :
   - H,
   - CH₃,
   - CH₂CH₃,
   - CH₂CH₂CₚF₂ₚ₊₁ avec p ≤ 4, où p est un entier,
   - CₘH₂ₘ₋₁ avec m ≤ 10, où m est un entier non nul, et
   - CₘH₂ₘ₊₁ avec m ≤ 10, où m est un entier non nul,
où un seul des radicaux R_{A} à R_{E} peut être un de ces deux derniers radicaux CₘH₂ₘ₋₁ et CₘH₂ₘ₊₁.

De préférence le radical R"' est une chaine alkyle, linéaire ou non, et en particulier un radical n-C₇H₁₅, n-C₉H₁₉, n-C₁₁H₂₃ ou n-C₁₃H₂₇.

Ces composés de type amide sont particulièrement adaptés au procédé d'extraction par différence de température selon l'invention. D'autres composés de ce type qui peuvent être utilisés en tant que MSA pour des compositions d'extraction sont par exemple :
le N-[3,5-Bis(trifluorométhyl)phényl]acétamide (No CAS 16143-84-3),
le N-[3,5-Bis(trifluorométhyl)phényl]-2-chloroacétamide (No CAS 790-75-0),
le N-[3,5-Bis(trifluorométhyl)phényl]-2-bromoacétamide (No CAS 99468-72-1),
le N-[3,5-Bis(trifluorométhyl)phényl]-2-chlorobenzamide (No CAS 56661-47-3),
le N-[3,5-Bis(trifluorométhyl)phényl]-4-chlorobenzamide (No CAS 56661-30-4),
le N-[3,5-Bis(trifluorométhyl)phényl]-4-bromobenzamide (No CAS 56661-31-5),
le N-[3,5-dichlorophényl]acétamide (No CAS 31592-84-4),
le N-[4-méthyl-3,5-dichlorophényl]acétamide (No CAS 39182-94-0),
le N-[3-fluoro-5-(trifluorométhyl)phényl]acétamide (No. CAS 402-02-8),
le N-[2-fluoro-5-(trifluorométhyl)phényl]acétamide (No. CAS 349-27-9),
le N-[4-chloro-3-(trifluorométhyl)phényl]acétamide (No. CAS 348-90-3),
le N-[4-bromo-3-(trifluorométhyl)phényl]acétamide (No. CAS 41513-05-7),
le N-[2,5-difluoro-3-(trifluorométhyl)phényl]acétamide (No. CAS 1994-23-6),
le N-[3-(trifluorométhyl)phényl]acétamide (No. CAS 351-36-0),
le N-[2-méthyl-3-(trifluorométhyl)phényl]acétamide (No. CAS 546434-38-2),
le N-[2-amino-3-(trifluorométhyl)phényl]acétamide (No. CAS 1579-89-1),
le N-[3-(trifluorométhyl)phényl]-2,2,2-trifluoroacétamide (No. CAS 2946-73-8),
le N-[3-(trifluorométhyl)phényl]-2,2-dichloroacétamide (No. CAS 2837-61-8),
le N-[3-(trifluorométhyl)phényl]-2,2,2-trichloroacétamide (No. CAS 1939-29-3),
le N-[4-chloro-3-(trifluorométhyl)phényl]-2,2,2-trichloroacétamide (No. CAS 13692-04-1),
le N-[3-(trifluorométhyl)phényl]-2-bromoacétamide (No. CAS 25625-57-4),
le N-[3-(trifluorméthyl) phényl]propanamide (No. CAS 2300-88-1),
le N-[2-chloro-5-(trifluorométhyl)phényl]propanamide (No. CAS 721-57-3),
le N-[3-(trifluorométhyl)phényl](2,2-diméthyl-propanamide) (No. CAS 1939-19-1),
le N-[2-methyl-3-(trifluorométhyl)phényl](2,2-diméthyl-propanamide) (No. CAS 150783-50-9),
le N-[4-chloro-2-methyl-3-(trifluorométhyl)phényl](2,2-diméthyl-propanamide) (No. CAS 112641-23-3),
le N-[3-(trifluorométhyl)phényl](2-chloro-propanamide) (No. CAS 36040-85-4),
le N-[3-(trifluorométhyl)phényl]butanamide (No. CAS 2339-19-7),
le N-[3-(trifluorométhyl) phényl]isobutanamide (No. CAS 1939-27-1),
le N-[3-(Trifluorométhyl)phényl]cyclopentanecarboxamide.(No. CAS 13691-84-4),
le N-[3-(trifluorométhyl)phényl](2-méthyl-pentanamide) (No CAS 1939-26-0),
le N-[3-(trifluorométhyl)phényl](2,2-Diméthyl-pentanamide) (No CAS 2300-87-0),
le N-[3-(trifluorométhyl)phényl](2-(4-Bromophényl)-acétamide) (No CAS 349420-02-6),
le N-[3-(Trifluorométhyl)phényl]-1-adamantanecarboxamide (No CAS 42600-84-0),
le N-[2-chloro-5-(trifluorométhyl)phényl]octanamide (No CAS 4456-59-1).

Ces molécules, employées en tant que MSA, par leur intégration dans une formulation combinant au moins un MEC et éventuellement un fluidifiant, permettent l'extraction d'espèces ioniques et en particulier de sels hydrophiles de l'eau vers la phase organique extractante.

### Concentration de MSA dans la composition liquide organique

Selon un aspect préféré de l'invention la concentration molaire du premier composé MSA (ou d'un mélange de tels composés) dans la composition selon l'invention est au moins égale à 0,1 M. De préférence cette composition est plus élevée, et est au moins égale à 1 M de manière à permettre une extraction optimisée en particulier des anions hydrophiles. Elle peut également être au moins égale à 2 M, avantageusement au moins égale à 3 M, par exemple au moins égale à 4 M. Dans certaines variantes de l'invention, le premier composé, ou un mélange de premier composés, peut être utilisé pur (concentration molaire de 7,32 M pour le n° CAS 349-75-7).

### Densité et solubilité et viscosité

Selon un aspect avantageux de l'invention, le premier composé permettant la solvatation, dans la composition, d'au moins un anion, a une solubilité à l'eau, sous sa forme libre ou complexée, inférieure à 0,1 Mol/L, de préférence inférieure à 0,01 Mol/L, de préférence inférieure à 0,0001 Mol/L et plus particulièrement inférieure à 1x10⁻⁵ Mol/L.

Selon un autre aspect avantageux de l'invention, le premier composé permettant la solvatation, dans la composition, d'au moins un anion présente une densité supérieure à 1,1 kg/L, idéalement supérieure à 1,2 kg/Litre.

Selon encore un autre aspect avantageux de l'invention, le premier composé permettant la solvatation, dans la composition, d'au moins un anion présente une viscosité à 25 °C de moins de 100 mPa.s, de préférence de moins de 50 mPa.s, par exemple inférieure à 20 mPa.s.

### MEC

Le deuxième composé, qui permet l'extraction d'au moins un cation (MEC), peut avantageusement être choisi parmi les molécules ayant une bonne capacité d'extraction des ions alcalins, comme par exemple des ions sodium, et/ou des ions alcalino-terreux ou d'autres cations suivant le besoin de séparation. L'extraction peut être du fait d'un remplacement de la solvatation des cations et anions par l'eau par une solvatation de ceux-ci par la composition extractante qui permet alors une intéraction avec les MEC et les MSA. La nature des interactions recouvre des phénomènes tels que des interactions ion-dipôle, accompagnées de l'établissement de liaisons hydrogènes et d'interactions électrostiques, voire de liaisons de van der Waals. De préférence la MEC est un composé permettant de complexer, et en particulier de chélater le cation. Le « Chélate » se distingue du simple *« complexe* » par le fait que le cation est fixé au ligand chélateur par au moins deux liaisons/interactions.

L'utilisation d'un éther couronne ayant un nombre de carbone pouvant aller de 14 à 80, en particulier des éthers couronnes non fluorés peut être considéré.

Par éther couronne on entend une molécule cyclique ayant un nombre de carbone pouvant aller de 14 à 80, on entend les éther couronne ayant 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 ou 80 atomes de carbone.

Ainsi le deuxième composé peut être choisi dans le groupe constitué du DB21C7, B15C5, C15C5, DC18C6, DB18C6, DB24C8, Calix[4]Est, et d'un calixarène substitué autre que le Calix[4]Est. La formule de ces composés étant indiquée ci-après.

Le deuxième composé peut être un calixarène substitué pouvant comprendre, par exemple, de 32 à 80 atomes de carbone, et plus particulièrement de 50 à 70 atomes, par exemple de 58 à 60 atomes de cabones. Le 4-tert-Butylcalix[4]arène-O,O',O",O‴-tetraacetic Acid Tetraethyl Ester est particulièrement préféré pour l'extraction du sodium.

La composition selon l'invention peut également comprendre plus d'un composé permettant l'extraction d'au moins un cation, celui-ci pouvant avantageusement être choisi dans les composés décrits dans la présente demande.

### Densité et solubilité et viscosité

Selon un aspect préférentiel de l'invention, le second composé permettant l'extraction, dans la composition, d'au moins un cation a une solubilité à l'eau, sous sa forme libre ou complexée au cation, inférieure à 0,1 Mol/L, de préférence inférieure à 0,01 Mol/L, de préférence inférieure à 0,0001 Mol/L et plus particulièrement inférieur à 1x10⁻⁵ Mol/L.

Selon un autre aspect préférentiel de l'invention, le second composé permettant l'extraction, dans la composition, d'au moins un cation présente une solubilité dans le premier composé (MSA), à 25°C supérieure à 0,2 M/L, de préférence supérieure à 0,5 M/L, par exemple supérieure à 1 M/L.

Selon un autre aspect préférentiel de l'invention, le second composé permettant l'extraction, dans la composition, d'au moins un cation présente une densité supérieure à 0,8 kg/L, de préférence supérieure à 1,0 kg/L, idéalement supérieure à 1,2 kg/L. Selon un autre aspect préférentiel de l'invention, le second composé permettant l'extraction dans le liquide d'au moins un cation est un liquide et présente une viscosité à 25 °C de moins de 100 mPa.s, de préférence de moins de 50 mPa.s, par exemple inférieure à 20 mPa.s.

### Concentration relative de MSA et de MEC dans la composition liquide organique

Pour assurer une extraction maximum des espèces ioniques, les concentrations de MSA et MEC sont choisies en fonction de la concentration dans la solution aqueuse des espèces ioniques à extraire.

Ainsi à iso-volume d'eau salée et de formulation d'extraction, la concentration en composé MEC est avantageusement équimolaire ou supérieure à la concentration du cation à extraire. Une concentration environs deux fois supérieure constituant généralement une limite au-delà de laquelle l'extraction des cations n'est pas substantiellement améliorée.

De manière surprenante une concentration molaire de MSA bien supérieure à celle de l'anion à extraire peut être requise pour effectuer une extraction optimisée. Ainsi au moins le double, de préférence le quadruple, voir le quintuple ou le sextuple, voir plus, de la concentration de l'anion à extraire peut-être nécessaire pour obtenir des résultats satisfaisants, en particulier lorsque l'anion est l'anion chlorure.

Ainsi la proportion relative molaire de MSA/MEC d'une composition selon l'invention pour extraire un sel constitué d'un anion et d'un cation est avantageusement supérieure ou égale à 1, 2, 3, 4, 5 ou 6. Le choix de la proportion relative molaire MSA/MEC à retenir pour une application industrielle est dépendant du cout relatif de ces composés et des données technico-économiques du projet. De préférence cette proportion est au moins égale à 4 pour un MSA Alcool et comprise entre 1 et 4 pour un MSAAmide.

### Utilisation de la composition selon l'invention

La composition selon l'invention peut être avantageusement utilisée pour extraire des ions (cations, anions) hydrophiles d'une phase aqueuse. Il convient de noter que cette extraction des ions n'est pas compensée par le transfert d'espèces chimiques, ioniques ou autre, de la phase organique à la phase aqueuse. Cette composition est particulièrement adaptée à l'extraction des espèces ioniques présentes dans de l'eau salée et en particulier l'eau de mer. Aussi, cette composition peut avantageusement être utilisée pour le dessalement d'eau de mer et généralement la purification ou le traitement des eaux salées. Par « salée » on entend une eau comprenant au moins un sel. Par « sel » on entend un composé ionique composé de cations et d'anions formant un produit neutre et sans charge nette. Ces ions peuvent être aussi bien inorganiques (chlorure Cl⁻, ion Na⁺...), qu'organique (acétate CH₃-COO⁻, ammonium R₃NH⁺...) et monoatomiques (fluorure F⁻, ion Mg²⁺...) aussi bien que polyatomiques (nitrates NO₃⁻, hydrogénocarbonate HCO₃⁻, sulfate SO₄²⁻...).

La composition selon l'invention est donc particulièrement apte à être utilisée dans un procédé où un dispositif d'extraction ionique.

### Anions et cations à extraire

Les premier et deuxième composés compris dans la composition selon l'invention sont des composés permettant la solvatation et l'extraction d'au moins une, et de préférence plusieurs, espèces ioniques constituant des sels alcalins ou alcalinoterreux. En particulier, ces espèces ioniques sont celles présentes dans de l'eau de mer et sont listées, ainsi que leurs concentrations respectives, dans le tableau II.

**Tableau II :**

| **Eau de mer** | **Mol/m³** | **g/m³-mg/L** |
|---|---|---|
| Br⁻ | 0,8 | 67,7 |
| Cl⁻ | 545,8 | 19349,8 |
| HCO₃⁻ | 1,8 | 108,2 |
| F⁻ | 0,1 | 1,4 |
| CO₃⁻⁻ | 0,3 | 15,8 |
| SO₄⁻⁻ | 28,2 | 2711,0 |
| Mg⁺⁺ | 52,4 | 1273,7 |
| Ca⁺⁺ | 10,3 | 412,0 |
| Sr⁺⁺ | 0,09 | 7,7 |
| Na⁺ | 469,3 | 10793,1 |
| K⁺ | 10,7 | 417,5 |
| | **1 120** | **35158** |

Aussi les compositions selon l'invention peuvent être utilisées dans des méthodes selon l'invention pour extraire dans une phase organique le Na⁺ ou le K⁺, ou un mélange de Na⁺ et de K⁺. De préférence, l'anion solvaté par la composition selon l'invention est un anion hydrophile, comme par exemple le Cl⁻ ou le SO₄²⁻ ou le HCO₃⁻ ou un mélange de Cl⁻ et de SO₄²⁻. Ainsi la composition selon l'invention est particulièrement adaptée à l'extraction d'une phase aqueuse de NaCl, de Na₂SO₄, de NaHCO₃, de KCI, de K₂SO₄ ou de KHCO₃, ou à l'extraction d'un mélange de NaCl et de Na₂SO₄, de NaCl et de NaHCO₃, de NaCl et de KCI, de NaCl et de K₂SO₄ ou de NaCl et de KHCO₃, ou de l'un quelconque de ces mélanges de sels, ou un mélange de NaCl et de Na₂SO₄ et de NaHCO₃ et de KCI et de K₂SO₄ et de KHCO₃.

Alternativement, ou additionnellement, les anions extraits sont des fluorures, des bromures, HCO₃⁻, nitrates NO₃⁻, CN⁻, OH⁻, nitrites NO₂⁻, carbonates CO₃²⁻, ou ClO₂⁻ ou sulfite SO₃²⁻ ou autres.

Pour les anions plus hydrophobes tels que les perchlorates ClO₄⁻, les permanganates MnO₄⁻, les picrates, de plus faibles concentrations de MSA suffisent pour effectuer leur transfert vers la phase organique en combinaison avec au moins un cation complexé par un MEC.

### FLUIDIFIANT

Certains des MEC et des MSA étant des composés solides ou visqueux aux températures de fonctionnement du procédé d'extraction, l'utilisation d'un fluidifiant est alors avantageuse. Comme, le procédé selon l'invention permet notamment d'extraire des concentrations relativement élevées de sels, identifier un solubilisant à même de dissoudre au moins 0,1 mol/L de MEC et de MSA, cumulés, doit être identifié. En effet, les solvants classiques tels que l'acétone, l'éthyle acétate, l'heptane, le diméthyl formamide, le nitrométhane, le méthanol, l'éthanol, le diéthyl éther ou l'acétonitrile par exemple ne solubilisent pas à ces niveaux de concentration nombre de MEC connus et en particulier le Calix[4]Ester, qui est un MEC d'intérêt.

Par contre, il apparait que des solvants tels que le chloroforme et plus particulièrement des solvants aromatiques polaires ont cette capacité d'être de bons candidats en tant que solubilisant pour cette application. Cela peut être expliqué par la nature semblable des MSA, eux même en général des composés aromatiques. Par exemple, le 1,3-bis(trifluorométhyl)benzène (n° CAS : 402-31-3) et plus préférentiellement le benzyle benzoate (n° CAS : 120-51-4) composés de deux cycles aromatiques répondent à ce critère de solubilisation sur des formulations testées intégrant le Calix[4]Ester et le MSA3. Ainsi la présence d'au moins un groupement trifluorométhyl électro-attracteur sur un aromatique ou 2 cycles aromatiques permettent d'obtenir des composés fluidifiant particulièrement avantageux.

Selon un aspect préférentiel de l'invention, la composition est constituée seulement des composés MSA et MEC, et éventuellement en association avec un composé fluidifiant constituant ainsi une composition constituée de MSA et MEC et d'un composé fluidifiant.

Selon un aspect préférentiel de l'invention, la composition ne comprend pas de composés classés comme dangereux et ne présente pas d'effet d'irritation cutané, est non allergisante et ne présente pas de toxicologie orale aigüe.

De préférence la composition selon l'invention ne contient pas de nitrobenzène.

Selon un mode de réalisation préféré la composition ne comprend pas de MEC permettant l'extraction d'ions calcium.

Contrairement à de nombreux procédés d'extraction déjà connus, le procédé selon l'invention n'est pas basé sur un changement de pH pour permettre soit l'absorption, soit le relargage des ions capturés, en particulier via une mobilité acido-basique de l'ion hydrogène H⁺. Ainsi un aspect préférentiel de l'invention est que le procédé ne comprend pas d'étape où le pH de l'eau de régénération liquide est significativement modifié, c'est-à-dire au-delà d'une variation de pH de +/- 2, par exemple de ± 1 par rapport à l'eau à traiter.

Le procédé étant particulièrement apte au dessalement d'eau de mer, les espèces ioniques considérées peuvent-être une de celles décrite au Tableau II ci-dessus. De plus ce procédé permet avantageusement d'extraire de l'eau à traiter, au moins une espèce cationiques alcaline ou alcalinoterreuse ainsi que des espèces anioniques telles que les ions Cl⁻ ou SO₄²⁻. Il convient de noter que de telles espèces anioniques sont hydrophiles et particulièrement difficiles à extraire d'un milieu aqueux. Un aspect particulièrement avantageux du procédé selon l'invention est qu'il peut permettre l'extraction d'une phase aqueuse de Na⁺, Cl⁻, SO₄²⁻, et de K⁺ de manière simultanée.

### ETAPE a)

L'étape de mélange a) de l'eau à traiter et de la phase organique peut être effectuée par agitation des deux phases liquides, par exemple par rotation, centrifugation, et/ou par interpénétration verticale (colonne gravitationnelle) lorsque ces deux phases sont de densités différentes. Ce dernier aspect est ce qui est préféré. Aussi, la phase organique est avantageusement choisie comme ayant une densité plus élevée que la densité de l'eau à traiter et de l'eau traitée. Alternativement, la phase organique peut être choisie comme ayant une densité moins élevée que la densité de l'eau à traiter et de l'eau traitée. Dans ces deux cas, le différentiel de densité doit être suffisant pour permettre une interpénétration effective des deux phases lorsque ce type de mélange est utilisé. Dans ce cas, ce différentiel est avantageusement d'au moins 0,1 kg/L. Cependant, si d'autres moyens de mélanges sont utilisés, tels que la centrifugation, alors ce différentiel peut n'être que d'au moins 0,05 kg/L.

Il est également préféré que l'étape de mélange a) ne se fasse pas dans des conditions aboutissant à une microémulsion ou à une émulsion stable.

### ETAPE b)

L'étape de séparation des phases aqueuses et organiques peut avantageusement être une simple décantation gravitationnelle de la phase organique et de la phase aqueuse liquide. Cette décantation peut avoir lieu dans le réacteur où s'effectue le mélange. Alternativement la séparation peut être obtenue par l'application d'un moyen extérieur, par exemple, la centrifugation, éventuellement dans une centrifugeuse distincte du réacteur où a lieu le mélange des phases aqueuse et organique.

### ETAPE c)

Une fois les phases séparées, la phase organique liquide chargée en espèces ioniques est dirigée vers le second réacteur où elle est mise en contact avec de l'eau liquide, où eau de régénération. A l'exception de la température, cette étape de mélange c) peut être effectuée dans des conditions opérationnelles similaires à celles décrites pour l'étape de mélange a). Cependant certaines des conditions, comme par exemple la pression, peuvent varier pour par exemple éviter une mise en ébullition de l'eau ou du fluidifiant.

### TEMPERATURE

Selon un aspect particulièrement avantageux de l'invention, l'étape a) est réalisée à température ambiante. Il est aussi avantageux que l'eau à traiter ne soit pas soumise à une étape de chauffage ou de refroidissement préalable. Alternativement, une étape de chauffage ou de refroidissement préalable peut avoir lieu. Dans ce cas il est préférable que l'eau à traiter ne soit chauffée ou refroidie de plus de 5 °C, avantageusement de plus de 2°C, par rapport à l'eau à traiter non chauffée ou non refroidie.

Selon un autre aspect avantageux de l'invention, la première température est à une température inférieure à 50°C mais avantageusement supérieure à 0°C. Cette température peut être choisie dans des gammes allant de 10°C à 40°C, de préférence de 15 °C à 30 °C, et particulièrement de 19 à 26 °C (par exemple 25°C).

Par gamme de température allant de 10 °C à 50°C, on entend des températures de 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C ou de 50 °C.

Selon un autre aspect avantageux de l'invention la deuxième température est une température supérieure à 50°C, de préférence supérieure à 70°C. Cette température peut être choisie dans des gammes allant de 50°C à 150°C, de préférence de 70°C à 110°C, et particulièrement de 80°C à 90°C (par exemple 85°C).

Par gamme de température allant de 50 °C à 150°C, on entend des températures de 50 °C, 51 °C, 52 °C, 53 °C, 54 °C, 55 °C, 56 °C, 57 °C, 58 °C, 59 °C, 60 °C, 61 °C, 62 °C, 63 °C, 64 °C, 65 °C, 66 °C, 67 °C, 68 °C, 69 °C, 70 °C, 71 °C, 72 °C, 73 °C, 74 °C, 75 °C, 76 °C, 77 °C, 78 °C, 79 °C, 80 °C, 81 °C, 82 °C, 83 °C, 84 °C, 85 °C, 86 °C, 87 °C, 88 °C, 89 °C, 90 °C, 91 °C, 92 °C, 93 °C, 94 °C, 95 °C, 96 °C, 97 °C, 98 °C, 99 °C, 100 °C, 101 °C, 102 °C, 103 °C, 104 °C, 105 °C, 106 °C, 107 °C, 108 °C, 109 °C, 110 °C, 111 °C, 112 °C, 113 °C, 114 °C, 115 °C, 116 °C, 117 °C, 118 °C, 119 °C, 120 °C, 122 °C, 124 °C, 126 °C, 128 °C, 130 °C, 132 °C, 134 °C, 136 °C, 138 °C, 140 °C, 142 °C, 144 °C, 146 °C, 148 °C ou de 150 °C.

Les premières et deuxièmes températures sont nécessairement choisies de manière à ce que le mélange reste à l'état liquide à la pression opératoire. Il est particulièrement avantageux que la différence entre ces températures, ΔT, soit choisie dans une gamme allant de 30 °C à 150 °C, de préférence de 50°C à 75°C. Par ΔT allant de 50 °C à 75°C, on entend un ΔT de 50 °C, 51 °C, 52 °C, 53 °C, 54 °C, 55 °C, 56 °C, 57 °C, 58 °C, 59 °C, 60 °C, 61 °C, 62 °C, 63 °C, 64 °C, 65 °C, 66 °C, 67 °C, 68 °C, 69 °C, 70 °C, 71 °C, 72 °C, 73 °C, 74 °C ou de 75 °C. Aussi, si la première température est de 20°C, la seconde température sera de plus de 50°C, avantageusement de plus de 70°C.

Ainsi, le procédé de l'invention peut comprendre une première étape a) permettant le transfert d'espèces ioniques de l'eau à traiter vers la phase organique, à température ambiante, suivie d'une étape c) permettant la régénération de la phase organique chargée en espèce ionique et qui a lieu à une température supérieure à la température ambiante mais relativement peu élevée (par exemple inférieure à 150 °C).

Selon un aspect préféré du procédé, il comprend les étapes subséquentes de :
d) séparation de ladite phase organique liquide régénérée et de l'eau liquide de régénération chargée en lesdites espèces ioniques,
e) mise en contact thermique indirecte, par exemple par échangeur de chaleur, de ladite phase organique liquide chargée en espèces ioniques et de ladite phase organique liquide régénérée.

Selon un aspect particulier de l'invention, il est avantageux que le procédé comprenne des étapes de chauffage et/ou de refroidissement de :
- la phase organique chargée en espèces ioniques,
- la phase organique, en particulier régénérée, non chargée en espèces ioniques,
- l'eau à traiter,
- l'eau traitée, et/ou
- de l'eau de régénération ;
qui précédent l'introduction de ces diverses phases ou eaux dans les premier et second réacteurs.

De telles étapes de chauffage peuvent être effectuées en tout ou partie par des échanges de chaleurs entre au moins deux des diverses phases précitées (c'est-à-dire les phases organiques et les phases aqueuse que sont l'eau à traiter, l'eau traitée et l'eau chargée en l'espèce ionique (saline)).

En particulier le procédé selon l'invention comprend une étape de chauffage de l'eau de régénération effectuée avant l'étape c).

### PRESSION

Les étapes de mélange a) et/ou c) sont avantageusement réalisées à la pression atmosphérique d'environ 1 atm au niveau de la mer, ou sans application de moyens de pression autres que le poids des liquides présents dans le réacteur.

Si une pression est appliquée, celle-ci peut être positive ou négative. Une telle pression peut aller de 0,8 atm à 80 atmosphères, de préférence de 1 à 10 atm.

### UTILISATION D'EAU TRAITEE

De manière avantageuse l'eau liquide de régénération utilisée à l'étape c) est une partie de l'eau traitée obtenue à l'issue de l'étape a). Alternativement elle peut être issue d'une source extérieure.

### COMPOSITION

La phase organique comprend, ou est essentiellement constituée, ou est constituée, de la composition selon l'invention qui est décrite dans la présente demande. Cette composition est particulièrement efficace pour mettre en œuvre ledit procédé. Des compositions particulièrement adaptées à la mise en œuvre du procédé selon l'invention comprennent des compositions MSA 7 et MSA 4 associées à des composés de types calixarène tel que le 4-tert-Butylcalix[4]arène-O,O',O",O‴-tetraacetic acid tetraethyl ester.

Dans la description de l'invention cette composition peut être également nommée « solvant » ou « résine ».

### DISPOSITIF

Le procédé selon l'invention peut être utilisé à l'aide d'un dispositif d'extraction d'au moins deux espèces ioniques, lesdites espèces ioniques comprenant au moins une espèce anionique et une espèce cationique, présentes dans de l'eau à traiter comprenant :
- un premier réacteur comprenant une phase ou composition organique hydrophobe liquide selon l'invention telle que décrite dans la présente demande.

Ce dispositif peut avantageusement comprendre :
- un premier réacteur comprenant ladite composition organique hydrophobe et liquide et éventuellement de l'eau à traiter, ladite eau à traiter étant à l'état liquide, pour l'obtention subséquente d'une eau traitée liquide et d'une phase organique liquide hydrophobe et chargée en lesdites espèces ioniques, ledit premier réacteur comprenant **en outre** des premiers moyens de mélange et des premiers moyens pour la séparation d'une part de ladite eau traitée liquide et d'autre part de ladite phase organique liquide chargée,
- un deuxième réacteur comprenant une phase organique hydrophobe liquide chargée en espèces ioniques et éventuellement de l'eau traitée liquide de régénération provenant dudit premier réacteur pour l'obtention subséquente d'une eau liquide de régénération chargée desdites espèces ioniques et d'une phase organique régénérée, ledit deuxième réacteur comprenant des seconds moyens de mélange et des seconds moyens pour la séparation d'une part de ladite eau liquide chargée en espèce ionique et d'autre part de ladite phase organique régénérée ;
- éventuellement des moyens de contrôle de la température dans ledit deuxième réacteur ;
- des moyens de communications permettant le transfert entre le premier et le second réacteur de :
   - ladite eau traitée liquide de régénération extraite dudit premier réacteur
   - ladite phase organique hydrophobe liquide chargée extraite dudit premier réacteur
   - ladite phase organique hydrophobe liquide régénérée extraite dudit second réacteur ; et, éventuellement,
   - un échangeur thermique mettant en présence d'une part ladite phase organique hydrophobe liquide chargée extraite dudit premier réacteur et d'autre part ladite phase organique hydrophobe liquide régénérée extraite dudit second réacteur et/ou ladite eau liquide chargée en espèce ionique.

Selon un aspect particulier, les réacteurs, et plus particulièrement les parties de ces réacteurs qui ne sont pas mobiles, ne sont pas en acier inoxydables.

Selon un autre aspect particulier le premier et/ou le deuxième réacteur ne comprend pas de moyens de chauffage (radiateurs) ou de refroidissement (réfrigérant).

Selon encore un autre aspect particulier la phase organique présente dans le dispositif comprend, ou est essentiellement constituée, ou est constituée de la composition décrite dans la présente demande.

Le dispositif peut avantageusement être monté en série pour permettre des étapes de traitement successives de l'eau à traiter de manière à diminuer la teneur en espèce ionique de l'eau jusqu'à obtenir une eau pure et/ou potable.

### DESCRIPTION DES FIGURES

L'invention sera mieux comprise à la lecture des figures annexées, qui sont fournies à titre d'exemples et ne présentent aucun caractère limitatif, dans lesquelles :
La Figure 1 est un graphique présentant les taux d'extraction de NaCl en % d'une eau salée à diverses concentrations (axe des abscisses) et à des températures (rond : 20°C, carré : 40°C, triangle 60°C et losange : 80°C) par utilisation d'une composition MSA 4/Calix[4]Est selon l'invention, à une concentration en MEC de 0,4M décrite à l'exemple 6B.
La Figure 2 est un graphique présentant, pour des compositions MSA 4/Calix[4]Est de l'exemple 6B (losange : 0,2M, rond : 0,4M et triangle 0,8M de MEC), les taux de chargement du MEC en NaCl en % pour diverses concentrations initiales en NaCl en Mol/L et à température ambiante.
La Figure 3 est un graphique présentant, les taux d'extraction en % de Na₂SO₄ d'une eau le contenant à diverses concentrations initiales et à température ambiante par utilisation d'une composition MSA 4/Calix[4]Est à diverses concentrations (triangle : 0,2M, losange : 0,4M et rond : 0,8M) selon l'invention décrite à l'EXEMPLE 6C.
La Figure 4 est une représentation schématique d'un exemple 7 de dispositif selon l'invention permettant de mettre en œuvre le procédé selon l'invention.
La Figure 5 est une autre représentation schématique de l'exemple de la Figure 4 indiquant un exemple de température de fonctionnement des diverses partie du dispositif.
La figure 6 est un tableau indiquant les concentrations de chaque espèce ionique dans chacun des flux identifiés dans le dispositif de l'exemple 7 ainsi que la salinité totale, la densité, la température et le débit de ces flux lorsque l'eau à traiter est de l'eau de mer.
La Figure 7 est une représentation schématique d'un autre exemple de dispositif selon l'invention décrit à l'exemple 8.
La Figure 8 une représentation schématique d'un autre exemple de dispositif selon l'invention décrit à l'exemple 9.
La Figure 9 représente le Spectre RMN du composé MSAC11

### EXEMPLES

### Exemple 1 : description des MEC testées

Différentes compositions extractrices d'ions selon l'invention ont été formulées et testées. Les 7 MEC utilisées dans ces compositions sont les suivantes :

| **Nom** | **Nomenclature** | **formule** |
|---|---|---|
| **EtherCoronne DB21C7** | 6, 7, 9, 10, 12, 13, 20, 21, 23, 24-Decahydrodibenzo[b,k] [1, 4, 7, 10, 13, 16, 19] heptaoxacyclohenicosine (Dibenzo-21-crown-7). CAS n° 14098-41-0, C₂₂H₂₈O₇, MW= 404 g/mole, MP = 107°C, S= 1,9 mMole/L (estimé à 25°C). | 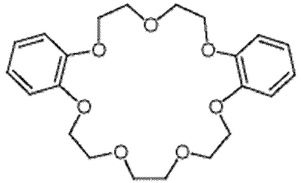 |
| | Log K(Na⁺, MeOH, 25°C) = 2,4 | |
| | Log K(K⁺, MeOH, 25°C) = 4,19 | |
| **EtherCoronne B15C5** | Benzo[b]-1,4,7,10,13-pentaoxa-cyclopentadecane (Benzo-15-Crown-5). | 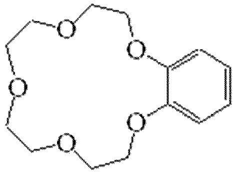 |
| | CAS n° 14098-44-3, C₁₄H₂₀O₅, MW= 268,31 g/mole, MP = 80°C, Log P = 0,91 (Exp), S= 11,6 mMole/L (estimé à 25°C). | |
| | Log K(Na⁺, MeOH, 25°C) = 3,03 | |
| | Log K(K⁺, MeOH, 25°C) = 3,93 | |
| **EtherCoronne C15C5** | Perhydrobenzo[b]-1,4,7,10,'3-pentaoxacyclopentadecane (Cyclohexo-15-Crown-5). | 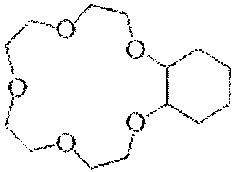 |
| | CAS n° 17454-48-7, C₁₄H₂₆O₅, MW= 274,35 g/mole, Liquide, FP = 100°C, d=1,12 g/mL, S = 57 mMole/L (estimé à 25°C). | |
| | Log K(Na⁺, MeOH, 25°C) = 3,71 - 3,9 | |
| | Log K(K⁺, MeOH, 25°C) = 3,96 | |
| **EtherCoronne DC18C6** | Dicyclohexano-1,4,7,10,13,16-hexaoxacyclooctadecane (Dicyclohexano-18-crown-6). | 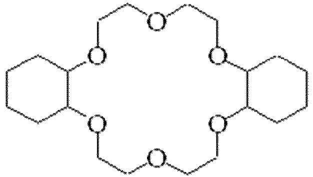 |
| | CAS n° 16069-36-6, C₂₀H₃₆O₆, MW= 372,51 g/mole, MP = 46-53°C, FP = 110°C, S = 36 mMole/L | |
| | Log K(Na⁺, MeOH, 25°C) = 4,27 | |
| | Log K(K⁺, MeOH, 25°C) = 5,97 | |
| **EtherCoronne DB18C6** | Dibenzo[b,k]-1,4,7,10,13,16-hexaoxacyclooctadecane (Dibenzo-18-crown-6). | 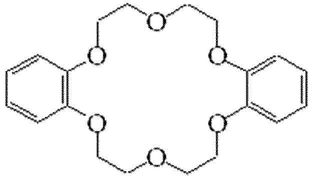 |
| | CAS n° 14187-32-7, C₂₀H₂₄O₆, MW= 360,41 g/mole, MP = 163°C, | |
| | Log P = 2,20 (Exp), S = 1,1 mMole/L | |
| | Log K(Na⁺, MeOH, 25°C) = 4,50 | |
| | Log K(K⁺, MeOH, 25°C) = 5,12 | |
| **EtherCoronne DB24C8** | 6,7,9,10,12,13,20,21,23,24,26,27-Dodecahydrodibenzo[b,n][1,4,7,10,13,16,1 9,22] octaoxacyclotetracosine (Dibenzo-24-crown-8). | 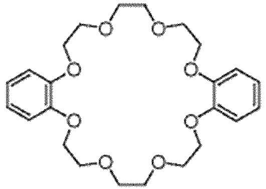 |
| | CAS n° 14174-09-5, C₂₄H₃₂O₈, MW= 448 g/mole, MP = 104°C, Log P = 2,11 (Exp), 1,865 mg/L (25°C estimé). | |
| | Log K(Na⁺, MeOH, 25°C) = 2,35 | |
| | Log K(K⁺, MeOH, 25°C) = 3,61 | |
| **Calixarene Calix[4]Est** | 4-tert-Butylcalix[4]arene-O,O',O",O‴-tetraacetic Acid Tetraethyl Ester (Calix[4]arene tetraesters, Sodium ionophore X). | 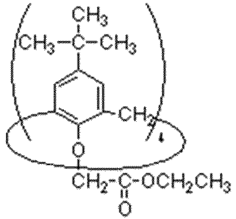 |
| | CAS n° 97600-39-0, C₆₀H₈₀O₁₂, MW= 993,27 g/mole, MP = 156-157°C, S < 1.10⁻⁶ mMole/L | |
| | Log K(Na⁺, MeOH, 25°C) = 5,0 | |
| **Cryptand DC[2.2.2]** | C₂₆H₄₈N₂O₆ (DiCyclohexanocryptand[222]) 5,6,14,15-DiCyclohexano-4,7,13,16,21,24-hexaoxa-1,10-diazabicyclo[8.8.8] hexacosane | 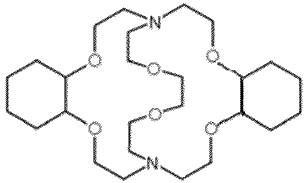 |
| | MW = 484 g/mole | |
| | S = 3 mMole/L | |
| | Log K(Na⁺, MeOH, 25°C) = 6,02 | |
| | Log K(K⁺, MeOH, 25°C) = 6,92 | |
| **Cryptand DB[2.2.2]** | C₂₆H₃₆N₂O₆ (Dibenzocryptand[222]) 5,6,14,15-DiBenzo-4,7,13,16,21,24-hexaoxa-1,10-diazabicyclo[8.8.8] hexacosane | 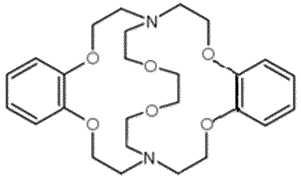 |
| | CAS : 40471-97-4 | |
| | MW = 472,57 g/mole | |
| | S = 2,5 mMole/L | |
| | Log K(Na⁺, MeOH, 25°C) = 7,60 | |
| | Log K(K⁺, MeOH, 25°C) = 8,74 | |
| **Cryptand Decyl[2.2.2]** | C₂₈H₅₆N₂O₆ (5-Decylcryptand[222]) 5-Decyl-4,7,13,16,21,24-hexaoxa-1,10-diazabicyclo[8.8.8]hexacosane | 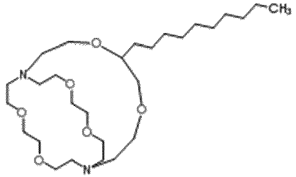 |
| | CAS : 69878-46-2 | |
| | MW = 516,75 g/mole | |
| | S = 0,1 mMole/L | |
| | Log K(Na⁺, MeOH, 25°C) = 7,04 | |
| | Log K(K⁺, MeOH, 25°C) = 9,0 | |

7 des 10 MEC présentées ci-dessus ont été solubilisées dans le SMA 1, puis des mesures d'extraction de NaCl ont été réalisées. Les autres ont été solubilisés dans le SMA 2.

### Exemple 2 : description des MSA testées

| **Paramètres** | **Valeurs** | **Unités** |
|---|---|---|
| Densité | 1,33 | kg/L |
| Viscosité | < 50 à 25°C | mPa.s |
| BP | 177-178 | °C |
| MP | -1,8 | °C |
| FP | 74 | °C |
| Log P | 2,95 | - |
| Solubilité | 3,83 (estimée) | mMole/L |
| pKa | 8,68 à 25°C | - |

**MSA 1** : **(3TFMPhOH)**
3-(Trifluoromethyl)phenol
N° CAS : 98-17-9
C₇H₅F₃O,
MW= 162,11 g/mole
Liquide incolore

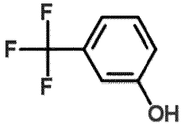

**MSA 2 : (3TFMBnOH) (Référence)**
[3-(Trifluoromethyl)phenyl]methanol
N° CAS : 349-75-7
C₈H₇F₃O,
MW= 176,14 g/mole
Liquide incolore et inodore.

| **Paramètres** | **Valeurs** | **Unités** |
|---|---|---|
| Densité | 1,295 | kg/L |
| Viscosité | 9,4 à 20°C | mPa.s |
| BP | 260 | °C |
| MP | < 25 | °C |
| FP | 84 | °C |
| Log P | 1,74 (estimée) | - |
| Solubilité | 32 | mMole/L |
| pKa | 14,74 +/- 1 | - |

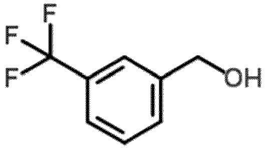

**MSA 3** : **(35TFMBnOH) (Référence)**
[3,5-Bis(Trifluoromethyl)phenyl]methanol
N° CAS : 32707-89-4
C₉H₆F₆O,
MW= 244,13 g/mole
Solide blanc.

| **Paramètres** | **Valeurs** | **Unités** |
|---|---|---|
| Densité | (1,433) | kg/L |
| Viscosité | - | mPa.s |
| BP | 255 | °C |
| MP | 55 | °C |
| FP | 97 | °C |
| Log P | 3,0 (estimée) | - |
| Solubilité | 2,29 | mMole/L |
| pKa | 14,7 +/- 1 | - |

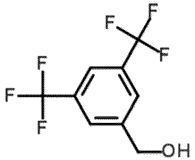

| **Paramètres** | **Valeurs** | **Unités** |
|---|---|---|
| Densité | 1,389 à 20°C | kg/L |
| Viscosité | 13,4 à 20°C | mPa.s |
| BP | - | °C |
| MP | < 15 | °C |
| FP | - | °C |
| Log P | 2,0 (estimée) | - |
| Solubilité | 15 | mMole/L |
| pKa | 14,7 +/- 1 | - |

**MSA 4 = 60%vol MSA 3+40%vol MSA 2 (Référence)**
Liquide blanc, incolore.

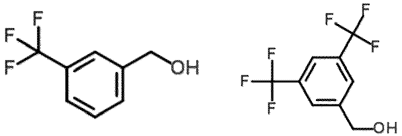

**MSA 5 : (3C4F9BnOH) (Référence)**
[3-(Perfluorobutyl)phényl]méthanol
N° CAS : Inconnu
C₁₁H₇F₉O,
MW= 326,16 g/mole
Liquide incolore et inodore.

| **Paramètres** | **Valeurs** | **Unités** |
|---|---|---|
| Densité | 1,488 | kg/L |
| Viscosité | 40 à 20°C | mPa.s |
| BP | à mmHg | °C |
| MP | < 15 | °C |
| FP | - | °C |
| Log P | 4,57 (estimé) | - |
| Solubilité | < 0,077 (non détecté en UV-visible) | mMole/L |
| pKa | 14,7 +/- 1 | - |

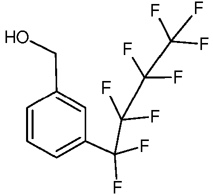

**MSA 6** : **(3,5-C3F7BnOH) (Référence)**
[3,5-(Perfluoropropyl)phenyl]methanol
N° CAS : Inconnu
C₁₃H₆F₁₄O,
MW= 444,16 g/mole

| **Paramètres** | **Valeurs** | **Unités** |
|---|---|---|
| Densité | 1,585 | kg/L |
| Viscosité | - | mPa.s |
| BP | à mmHg | °C |
| MP | - | °C |
| FP | - | °C |
| Log P | 5,75 (estimé) | - |
| Solubilité | < 0,07 | mMole/L |
| pKa | 14,25 +/- 1 | - |

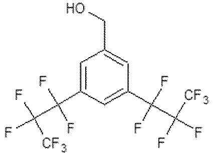

Dans les tableaux de données ci-dessus les acronymes BP, MP et FP désignent :
BP (boiling point) = température d'ébullition
MP (melting point) = point de fusion
FP (flash point) = point éclair

### Exemple 3

Le composé de formule MSA5 a été synthétisé de la manière suivante :

### Première étape

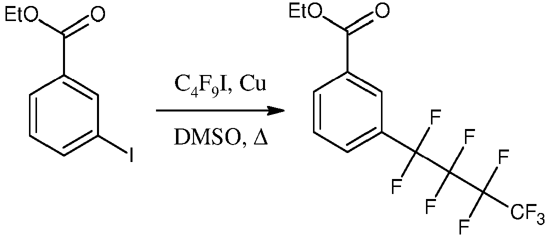

Une solution de 3-iodobenzoate d'éthyle (207.9 g, 753.2 mmol, 1.0 éq.), de cuivre en poudre (239.3 g, 3.766 mol, 5.0 éq.) et de 450 mL de DMSO est dégazée puis mise sous atmosphère d'argon. Le mélange est ensuite porté à 130 °C puis une solution de 1-iodoperfluorobutane (181.5 mL, 1.054 mol, 1.4 éq) est ajoutée goutte à goutte en 30 minutes. Le mélange réactionnel est agité à 130 °C pendant 5 h sous atmosphère d'argon. Après retour à température ambiante, 2 L d'acétate d'éthyle et 1 L d'eau sont ajoutés. Le mélange est ensuite filtré sur silice (Célite). La phase organique est lavée à l'eau (2 x 1 L), séchée sur sulfate de sodium, filtrée puis concentrée sous pression réduite pour donner l'éthyle 3-(perfluorobutane)benzoate brut (269.0 g, 730.6 mmol, 97 %, liquide marron clair).

¹H RMN (CDCl₃, 300 MHz) : δ (ppm) = 1.42 (t, *³J* = 7.1 Hz, 3H), 4.44 (q, *³J* = 7.1 Hz, 4H), 7.61 (t, *³J* = 8.0 Hz, 1H), 7.78 (d, *³J* = 7.7 Hz, 1H), 8.24-8.30 (m, 2H).

### Deuxième étape

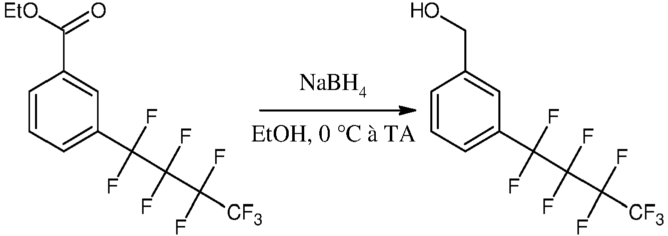

A une solution refroidie à l'aide d'un bain de glace de 3-(perfluorobutane)benzoate d'éthyle (269.0 g, 730.6 mmol, 1.0 éq.) et de 500 mL d'éthanol est additionnée par petites portions du borohydrure de sodium (82.9 g, 2.192 mol, 3.0 éq.). La température est contrôlée et doit être inférieure à 20 °C. Une fois l'addition terminée, le mélange réactionnel est agité à température ambiante pendant 15 h. Une fois l'agitation terminée, une solution saturée de NH₄Cl (2 L) est additionnée à froid puis dilué avec 2 L d'acétate d'éthyle. La phase aqueuse est extraite à l'acétate d'éthyle (1 x 1 L), puis les phases organiques sont lavées avec i) une solution saturée de NH₄Cl (1 x 1 L) et ii) à l'eau (1 x 1 L). Après séchage sur sulfate de sodium et filtration, la phase organique est concentrée sous pression réduite pour donner le (3-perfluorobutyl)phenylméthanol brut (228.9 g, 701.8 mmol, 96 %, liquide marron clair).

Le composé brut est purifié par distillation sous vide (P = 5 mmbars, T_{eb} = 98-102 °C) pour donner le (3-perfluorobutyl)phenylméthanol (162.5 g, 498.2 mmol, 68 %, liquide incolore).

¹H RMN (CDCl₃, 300 MHz) : δ (ppm) = 1.72 (br s, 1H), 4.79 (s, 2H), 7.49-7.53 (m, 2H), 7.56-7.62 (m, 2H).

### Exemple 4

Le composé de formule MSA6 a été synthétisé de la manière suivante :

### Première étape

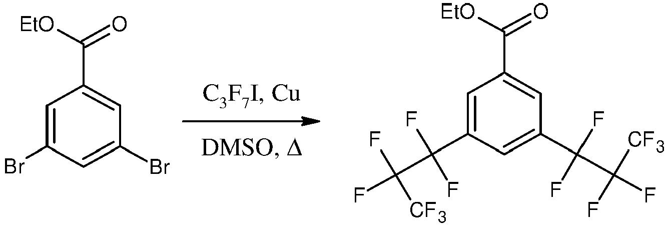

Quelques cristaux d'iode sont ajoutés à une suspension de cuivre en poudre (10.32 g, 162.4 mmol, 5.0 éq.) et d'acétone (50 mL). Après 30 min d'agitation, la phase liquide est éliminée par filtration et le cuivre est lavé par une solution d'acide chlorhydrique gazeux dans l'acétone (60 mL) puis à l'acétone (60 mL). Le cuivre activé est introduit à une solution de 3,5-dibromobenzoate d'éthyle (10.0 g, 32.5 mmol, 1.0 éq.) et de 500 mL de DMSO. La suspension est dégazée puis mise sous atmosphère d'argon. Le mélange est ensuite porté à 130 °C. Une solution de 1-iodoperfluoropropane (13.2 mL, 90.9 mmol, 2.8 éq) est ajoutée goutte à goutte en 30 minutes. Le mélange réactionnel est agité à 130 °C pendant 5 h sous atmosphère d'argon. Après retour à température ambiante, 50 mL d'acétate d'éthyle et 50 mL d'eau sont ajoutés. Le mélange est ensuite filtré sur Célite. La phase organique est lavée à l'eau (2 x 50 mL), séchée sur sulfate de sodium, filtrée puis concentrée sous pression réduite pour donner l'éthyle 3,5-bis(perfluoropropane)benzoate brut (15.46 g, 31.8 mmol, 98 %, solide jaune).

¹H RMN (CDCl₃, 300 MHz) : δ (ppm) = 1.45 (t, *³J* = 7.1 Hz, 3H), 4.46 (q, *³J* = 7.1 Hz, 4H), 7.96 (br. s, 1H), 8.48 (br. s, 2H).

### Deuxième étape

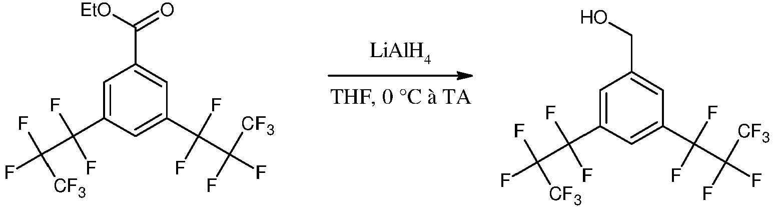

Une solution d'éthyle 3,5-bis(perfluorobutane)benzoate (15.46 g, 31.8 mmol, 1.0 éq.) et de 100 mL de THF anhydre est additionnée goutte à goutte à une suspension de LiAlH₄ (1.81 g, 47.7 mmol, 1.5 éq.) et de THF anhydre (10 mL) sous atmosphère d'argon et à 0 °C. Une fois l'addition terminée, le mélange réactionnel est agité à température ambiante (TA) pendant 5 h. Ensuite, 10 mL d'acétate d'éthyle sont ajoutés très lentement. Après 15 min, 10 mL d'une solution d'acide sulfurique 10 % est ajoutée avec précaution à 0 °C, puis le milieu réactionnel est agité pendant 20 min. La phase aqueuse est extraite avec de l'acétate d'éthyle (3 x 50 mL). Les phases organiques sont réunies, lavées avec une solution saturée de NaCl (1 x 50 mL), séchées sur sulfate de magnésium, filtrées puis concentrées sous pression réduite pour donner un solide jaune pâle. Ce solide est recristallisé dans l'hexane pour donner le [3,5-bis(perfluorobutyl)]phenylméthanol (12.95 g, 29.3 mmol, 92 %, liquide).

¹H RMN (CDCl₃, 300 MHz) : δ (ppm) = 4.88 (s, 2H), 7.70 (br. s, 1H), 7.82 (br. s, 2H).

### Exemple 5 : Compositions comprenant une MSA fluorée de type phénolique : 3TFMPhOH avec différentes MEC

Le 3-(Trifluoromethyl)phenol a été acheté auprès de AlfaAesar, et a une pureté de 98+%. Il a été employé en l'état.

Le Dibenzo-18-crown-6 a été acheté auprès de TCI Chemicals, et a une pureté de >99%, il a été employé en l'état.

A 3 mL de 3-(Trifluoromethyl)phenol ont été ajoutés 217 mg de Dibenzo-18-crown-6 afin d'obtenir une formulation à 0,2 mole/L de DB18C6. Cette formulation scellée a ensuite été mise en agitation orbitalaire à 500 tours/minute pour la nuit après avoir ajouté 1 mL d'eau distillée deux fois afin de permettre une saturation à l'eau de l'ensemble.

Le lendemain matin, on prépare une solution aqueuse de NaCl à 0,2 mol/L à partir d'une eau distillée deux fois, pendant que la formulation ayant été agitée la nuit est mise au repos pour décantation. Une décantation nette des deux phases incolores est obtenue en quelques minutes. 3 mL de la solution organique d'extraction sont alors prélevés et transvasés vers un flacon contenant 3 mL de cette eau salée comprenant du NaCl à 0,2 M, puis le flacon est scellé et mis sous agitation orbitalaire (en général à 500 tours par minutes), pendant 3 heures et à température ambiante. Il est vérifié que des gouttelettes de l'ordre de 1-2 mm sont bien présentes en quantité à la vitesse d'agitation choisie (400 à 900 tours/min).

Une fois les 2 heures d'agitation réalisées, l'agitation est arrêtée et l'ensemble est mis au repos pour décantation pendant 10 minutes au moins jusqu'à séparation totale des deux phases. Puis, la phase aqueuse supérieure est prélevée puis agitée et diluée pour analyse de sa salinité par une Chromatographie Ionique de marque Metrohm intégrant une colonne d'analyse des cations, adaptée et une colonne d'analyse des anions, adaptée. De même, la solution aqueuse initiale de NaCl à 0,2M est également analysée par cette chromatographie ionique pour déterminer sa concentration relative en sodium et en chlorures.

### RESULTATS :

| MSA: **3TFMPhOH** | MEC : **DB18C6 0,2M** | [Na+] mmol/L | [CI-] mmol/L | Moyenne mmol/L |
|---|---|---|---|---|
| Eau à traiter 0 | **NaCl 0,2M** | 188,77 | 193,33 | 191,05 |
| Eau traitée | NaCl 0,125M | 130,6 | 119,4 | 125,0 |
| Taux d'extraction à **23°C** | ([NaCl]_{aq0} - [NaCl]_{aq}) / [NaCl]_{aq0} | 30,8% | 38,8% | **34,6%** |

Cette composition est capable d'extraire par contact direct, à iso-volume et à température ambiante un peu plus d'un tiers du NaCl présent dans l'eau. De plus, un peu plus d'un tiers des molécules extractantes du sodium sont sous forme complexées. Ainsi, grâce à la présence du MSA, nous observons un 34,6% d'extraction, là où l'on ne dépasse pas les 1,6% en remplaçant ce MSA par du dichlorométhane.

Le même mode opératoire que décrit ci-dessus pour le DB18C6 a été appliqué aux autres MEC décrites à l'Exemple 1. Les résultats de l'analyse chromatographique sont les suivants :

| MSA: **3TFMPhOH** | MEC : **DB21C7 0,2M** | [Na+] mmol/L | [CI-] mmol/L | Moyenne mmol/L |
|---|---|---|---|---|
| Eau à traiter 0 | **NaCl 0,2M** | 183,77 | 196,29 | 190,03 |
| Eau traitée | NaCl 0,16M | 166,4 | 155,7 | 161,1 |
| Taux d'extraction à **23°C** | ([NaCl]_{aq0} - [NaCl]_{aq}) / [NaCl]_{aq0} | 9,4% | 20,7% | **15,2%** |

| MSA : **3TFMPhOH** | MEC : **B15C5 0,2M** | [Na+] mmol/L | [CI-] mmol/L | Moyenne mmol/L |
|---|---|---|---|---|
| Eau à traiter 0 | **NaCl 0,2M** | 188,77 | 193,33 | 191,05 |
| Eau traitée | NaCl 0,16M | 156,4 | 169,1 | 162,7 |
| Taux d'extraction à **23°C** | ([NaCl]_{aq0} - [NaCl]_{aq}) / [NaCl]_{aq0} | 17,2% | 12,5% | **14,8%** |

| MSA: **3TFMPhOH** | MEC : **C15C5 0,2M** | [Na+] mmol/L | [CI-] mmol/L | Moyenne mmol/L |
|---|---|---|---|---|
| Eau à traiter 0 | **NaCl 0,2M** | 183,77 | 196,29 | 190,03 |
| Eau traitée | NaCl 0,14M | 145,2 | 136,0 | 140,6 |
| Taux d'extraction à **23°C** | ([NaCl]_{aq0} - [NaCl]_{aq}) / [NaCl]_{aq0} | 21,0% | 30,7% | **26,0%** |

| MSA: **3TFMPhOH** | MEC : **DC18C6 0,2M** | [Na+] mmol/L | [CI-] mmol/L | Moyenne mmol/L |
|---|---|---|---|---|
| Eau à traiter 0 | **NaCl 0,2M** | 192,45 | 182,55 | 187,50 |
| Eau traitée | NaCl 0,13M | 136,4 | 127,2 | 131,8 |
| Taux d'extraction à **23°C** | ([NaCl]_{aq0} - [NaCl]_{aq}) / [NaCl]_{aq0} | 29,1% | 30,3% | **29,7%** |

| MSA: **3TFMPhOH** | MEC : **DB24C8 0,2M** | [Na+] mmol/L | [CI-] mmol/L | Moyenne mmol/L |
|---|---|---|---|---|
| Eau à traiter 0 | **NaCl 0,2M** | 192,45 | 182,55 | 187,50 |
| Eau traitée | NaCl 0,12M | 124,0 | 114,9 | 119,5 |
| Taux d'extraction à **23°C** | ([NaCl]_{aq0} - [NaCl]_{aq}) / [NaCl]_{aq0} | 35,5% | 37,1% | **36,3%** |

| MSA: **3TFMPhOH** | MEC : **Calix[4]Est 0,2M** | [Na+] mmol/L | [CI-] mmol/L | Moyenne mmol/L |
|---|---|---|---|---|
| Eau à traiter 0 | NaCl 0,2M | 192,45 | 182,55 | 187,50 |
| Eau traitée | NaCl 0,045M | 48,1 | 41,9 | 45,0 |
| Taux d'extraction à **23°C** | ([NaCl]_{aq0} - [NaCl]_{aq}) / [NaCl]_{aq0} | 75% | 77% | **76%** |

Ces résultats montrent que le taux d'extraction du NaCl est fortement dépendant de l'affinité du MEC, c'est à dire de l'extractant, pour le cation Na⁺.

En fait, en prenant en compte les constantes de complexation publiées pour l'ensemble de ces extractants MEC pour le sodium, dans le méthanol à 25°C, il est observé une corrélation, linéaire en première partie, puis, **de manière surprenante,** plus fortement croissante à partir du moment où l'affinité du MEC pour le sodium dans l'eau dépasse un Log K de 1. De plus cette même tendance est également obtenue en extraction du KCI ou du Na₂SO₄.

| **MEC** | DB21C7 | B15C5 | C15C5 | DC18C6 | DB18C6 | Calix4Est | DB24C8 |
|---|---|---|---|---|---|---|---|
| **Log K (Na+) MeOH à 25°C** | 2,4 | 3,03 | 3,71-3,9 | 4,27 | 4,36-4,49 | 5,0-5,7 | 2,25 |
| **Taux d'extraction** | 15,2% | 14,8% | 26,0% | 29,7% | 34,6% | 76% | 36,3% |

Seul le DB24C8 semble ne pas respecter cette règle. Une explication pourrait venir du fait que cet éther couronne est très large. En effet, il a déjà été observé une absorption de deux cations Na+ par ces macrocycles. Or la constante de complexation de 2,25 de ce composé DB24C8 correspondrait au cas où un seul cation est complexé. Une constante de complexation deux fois plus élevée que celle publiée pour DB24C8 (c'est-à-dire de 4,5) rétablirait alors la corrélation susmentionnée. Ainsi une MEC présentant à la fois :
- une constante de complexation pour le sodium, dans l'eau à 25°C, supérieure ou égale à 1, et
- une constante de complexation pour le sodium, dans l'éthanol à 25°C, supérieure ou égale à 4, de préférence supérieure à 4,75, permet des taux d'extraction ionique particulièrement élevés, et notamment pour des sels tels que NaCl, KCI ou du Na₂SO₄.

### EXEMPLE 6 DE REFERENCE : Compositions comprenant une MSA fluorée de type phényle méthanolique (MSA 2 et 5) ou un mélange de ces composés (MSA 4 et 7) avec la MEC : Calix[4]Est

### EXEMPLE 6A DE REFERENCE : Composition MSA 2 / Calix[4]Est pour l'extraction de NaCl

Le composé MSA 2 a été acheté chez Fluorochem (97% de pureté) et utilisé en l'état.

La composition MSA 2 / Calix[4]Est est préparée, testée et analysée selon le même protocole que celui décrit à l'Exemple 5 précédent.

Résultats pour la composition MSA 2/ Calix[4]Est :

| MSA : **TFMBnOH** | MEC : **0,2M Calix[4]Est** | [Na+] mmol/L | [Cl-] mmol/L | Moyenne mol/L |
|---|---|---|---|---|
| Eau à traiter 0 | NaCl 0,2M | 176,96 | 208,34 | 192,65 |
| Eau traitée | NaCl 0,08M | 80,8 | 78,8 | 79,8 |
| Taux d'extraction à **23°C** | ([NaCl]_{aq0} - [NaCl]_{aq}) / [NaCl]_{aq0} | 54,3% | 62,2% | **58,6%** |

### EXEMPLE 6B DE REFERENCE : Composition MSA 4 / Calix[4]Est pour l'extraction de NaCl

Le MSA 4 est un mélange de MSA 2 et de MSA 3 (solide aux conditions normales de température et de pression). 30,4 mL de MSA 4 ont été formulés en ajoutant 12,16 mL de MSA 2 à 26,14 g de MSA 3. Puis, après agitation et dissolution de MSA 2, 6,04 g de Calix[4]Est ont été ajoutés et solubilisés rapidement au moyen d'un léger chauffage à 40°C. Une dilatation de la formulation est observée après solubilisation du Calix[4]Ester et saturation en eau. Ces compositions ont été testées et analysées selon le même mode opératoire que celui décrit à l'Exemple 5.

Résultats pour la composition MSA 4/ Calix[4]Est :

| **MSA 4:3TFMBnOH + 35TFMBnOH** | MEC **:Calix[4]Est 0,2M** | [Na+] mmol/L | [CI-] mmol/L | Moyenne mmol/L |
|---|---|---|---|---|
| Eau à traiter 0 | **NaCl 0,2M** | 212,71 | 205,48 | 209,09 |
| Eau traitée | NaCl 0,04M | 42,1 | 46,5 | 44,3 |
| Taux d'extraction à **23°C** | ([NaCl]_{aq0} - [NaCl]_{aq}) / [NaCl]_{aq0} | 80,2% | 77,4% | **78,8%** |

Cet exemple particulier a été reproduit une seconde fois pour donner une performance moyenne d'extraction à 78,9%, donc concordante.

La présence d'un second trifluorométhyle en méta de la fonction alcool a un effet très favorable sur l'extraction du NaCl en permettant une meilleure solvatation des anions.

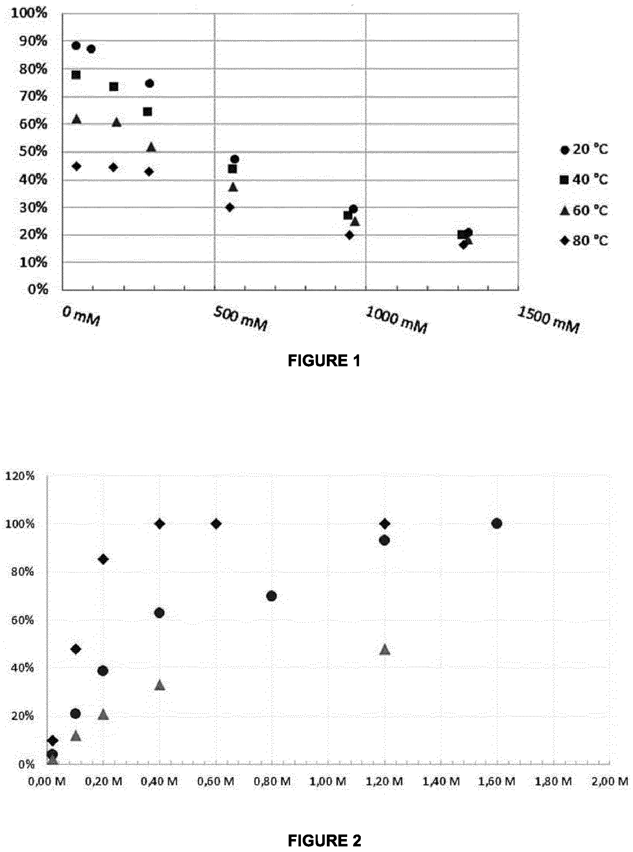

Le graphique de la Figure 1 représente les taux d'extraction obtenus par une composition MSA 4 et Calix4Est (0,4 M) pour de l'eau salée (NaCl) de diverses salinités et à des températures variables allant de la température ambiante à 80°C et à iso-volume Eau/Composition (MSA 4/ Calix[4]Est).

La performance d'extraction est tout à fait remarquable avec des taux d'extraction du NaCl allant de 90% pour les plus basses concentrations à 15% pour les concentrations les plus élevées, le tout à iso-volume eau/solvant avec une baisse du taux d'extraction de l'ordre d'un tiers dès 60°C par rapport à 20°C.

Pour cette composition MSA 4/Calix[4]Est, il est calculé via ces résultats que les interactions enthalpiques misent en œuvre sont de l'ordre de 33kJ/mole de sels déplacés. Ainsi, pour un déplacement de 36 g de NaCl par litre d'eau (concentration de l'eau de mer standard) une énergie de base de seulement 21 kJ/kg d'eau dessalée est ainsi nécessaire. La chaleur latente de vaporisation de l'eau étant de 2319 kJ/kg à 75°C, l'énergie consommée lors de la mise en œuvre du procédé selon l'invention est 100 fois moindre que celle nécessaire à l'évaporation de l'eau.

Le graphique de la FIGURE 2 représente le taux de chargement du MEC, Calix[4]Est en NaCl, en fonction de la concentration en MEC (losange : 0,2M, rond : 0,4M et triangle 0,8M), lors de son extraction d'eaux salées de diverses concentrations. Pour obtenir un taux de chargement optimal (proche de 100%) en fin de phase d'extraction/d'absorption pour de l'eau salée à des salinités proches de la salinité typique de l'eau de mer (0,6 M), une concentration comprise entre 0,2 M et 0,4 M de Calix[4]Est est à préférer.

### EXEMPLE 6C DE REFERENCE : Composition MSA 4/Calix[4]Est pour l'extraction de Na₂SO₄ :

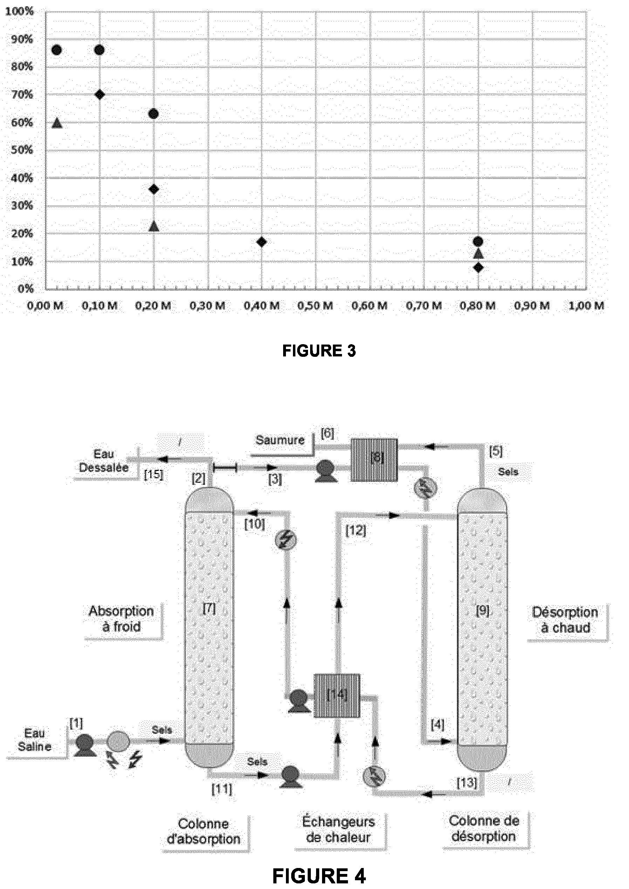

L'extraction du Na₂SO₄ a également été réalisée avec la composition MSA 4/Calix[4]Est précédemment décrite et à diverses concentrations de Calix[4]Est (triangle: 0,2M, losange : 0,4M et rond 0,8M). Le graphe de la Figure 3 représente les taux d'extractions de ce sel qui ont été obtenues pour des eaux de différentes concentrations en Na₂SO₄ suivant le protocole précédemment décrit. Il apparaît qu'une plus grande concentration de MEC permet une extraction de Na₂SO₄ améliorée.

Bien que les sulfates appartiennent aux anions les plus hydrophiles, nous observons encore une fois une bonne extraction de ces sels sur l'ensemble des concentrations testées.

### EXEMPLE 6D DE REFERENCE : Composition MSA 5/Calix[4]Est pour l'extraction de NaCl :

Le composé MSA 5 a été synthétisé suivant le procédé décrit à l'Exemple 3 et utilisé en l'état.

La composition MSA 5 / Calix[4]Est est préparée, testée et analysée selon le même protocole que celui décrit à l'Exemple 5 excepté que l'agitation orbitalaire utilisée a été de 900 trs/min en raison d'une plus grande viscosité de cette formulation.

Résultats pour la composition MSA 5/ Calix[4]Est:

| MSA 5: 3C4F9BnOH | MEC :Calix[4]Est 0,2M | [Na+] mmol/L | [CI-] mmol/L | Moyenne mmol/L |
|---|---|---|---|---|
| Eau à traiter 0 | NaCl 0,2M | 189,17 | 191,24 | 190,20 |
| Eau traitée | NaCl 0,086M | 86,65 | 86,44 | 86,55 |
| Taux d'extraction à 23°C | ([NaCl]_{aq0}-[NaCl]_{aq}) / [NaCl]_{aq0} | 54,2% | 54,8% | 54,5% |

Il est obtenu un taux d'extraction de NaCl un peu moins bon que MSA 2 mais pour un produit à solubilité à l'eau très inférieure (< 0,077 contre 32 mMole/L).

### EXEMPLE 6E DE REFERENCE : Composition MSA 7 / Calix[4]Est pour l'extraction de NaCl

Le MSA 7 est un mélange de 70 % MSA 5 et de 30 % MSA 6 v/v. Il a été obtenu selon le même procédé que selon utilisé pour le MSA 4 après recalcule des masses de produits à mettre en présence.

Ces compositions ont été synthétisées, formulées, testées et analysées selon le même mode opératoire que celui décrit à l'Exemple 5.

Résultats pour la composition MSA 7/ Calix[4]Est :

| MSA 7: 3C4F9BnOH +35C3F7BnOH | MEC : Calix[4]Est 0,2M | [Na+] mmol/L | [CI-] mmol/L | Moyenne mmol/L |
|---|---|---|---|---|
| Eau à traiter 0 | NaCl 0,2M | 212,7 | 205,5 | 209,1 |
| Eau traitée | NaCl 0,05M | 54,2 | 50,2 | 52,2 |
| Taux d'extraction à 23°C | ([NaCl]_{aq0} - [NaCl]_{aq}) / [NaCl]_{aq0} | 74,5% | 75,6% | 75% |

Il est obtenu un taux d'extraction de NaCl un peu moins bon que MSA 4 mais pour un produit à solubilité à l'eau très inférieure (< 0,07 contre 15 mMole/L).

### EXEMPLE 7 Procédé et Dispositif

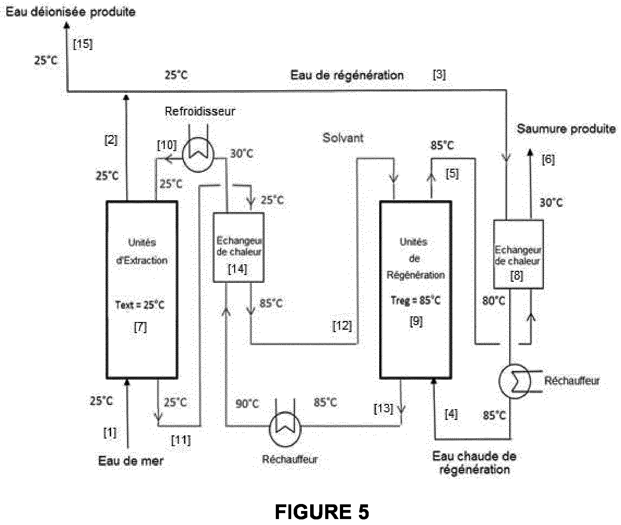

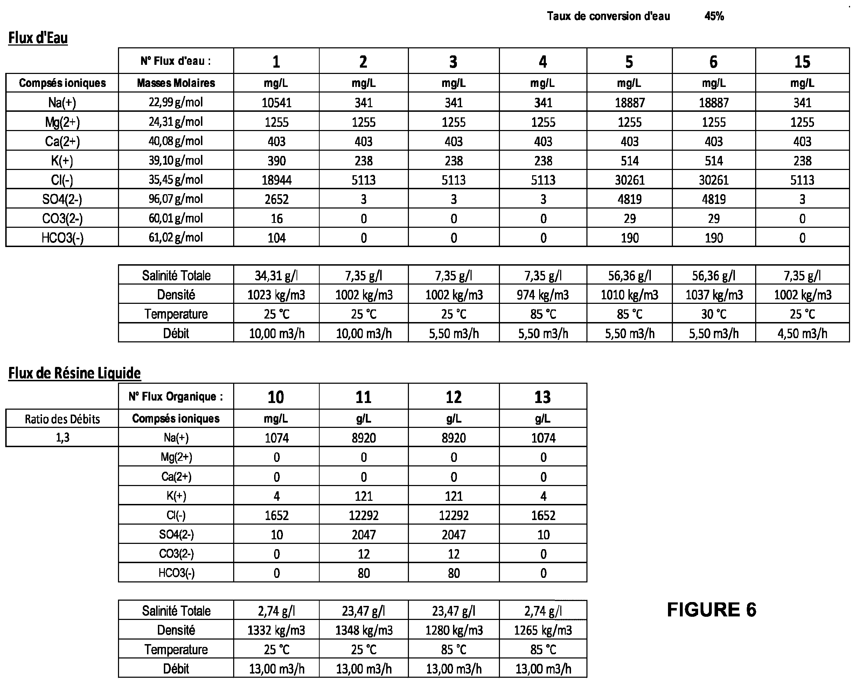

Un exemple de dispositif permettant de mettre en œuvre le procédé selon l'invention est présenté en Figures 4 et 5. Cet exemple porte sur un système d'extraction/d'absorption d'ions à froid associé à un système de désextraction/désorption d'ions à chaud, tous deux en phase liquides. La Figure 5 comprend une indication des températures des liquides à chaque étape et pour chaque flux du dispositif. La Figure 6 est un tableau indiquant les concentrations de chaque espèce ionique dans chacun des flux identifiés ainsi que la salinité totale, la densité, la température et le débit de ces flux lorsque l'eau à traiter est de l'eau de mer.

### REACTEUR

Le dispositif comprend un premier réacteur (7) et un deuxième réacteur (9) permettant le mélange de la phase organique et de la phase aqueuse et la décantation des liquides. Ce mélange permet le contact entre les deux phases et donc l'échange d'ions. Plus le contact est intime plus l'échange d'ions est important.

De tels réacteurs (7) et (9) peuvent comprendre des colonnes gravitationnelles d'extraction/d'absorption liquide-liquide (tel que représentées à la Figure 4 où les réacteurs (7) et (9) sont avantageusement de construction similaire). Alternativement le réacteur (7) et/ou le réacteur (9) peut être choisi de type mélangeur-décanteur et/ou extracteur/décanteur centrifuge.

Ces réacteurs (7) et (9) peuvent ainsi comprendre des moyens d'agitation (par exemple au moins un agitateur) permettant au mélangeur d'assurer une meilleure action de pompage par écoulement axial ou radial et une action de turbulence avec un cisaillement plus ou moins élevé.

Ces moyens d'agitation comprennent des éléments mobiles, tels que des hélices ou d'autres éléments rotatifs de cisaillement et/ou de turbulence. Ils peuvent également comprendre des moyens de centrifugation et/ou une centrifugeuse, par exemple comprendre un décanteur centrifuge.

Alternativement ou cumulativement ils peuvent comprendre des moyens de cisaillement statiques, tels que la présence à l'intérieur du réacteur de garnissages, organisés ou non, agissant en tant que butée venant s'opposer à la progression du liquide et résultant dans l'effet de turbulence et/ou de cisaillement du liquide présent au sein du réacteur.

### PROCEDE : TRAITEMENT DE L'EAU

Dans l'exemple représenté aux figures 4 et 5, la colonne d'absorption (7) permet donc le mélange d'une phase liquide organique hydrophobe selon l'invention qui est choisie de sorte qu'elle présente une densité plus élevée que l'eau à traiter et que la saumure produite.

Ainsi, lorsque le réacteur est une colonne, les deux phases liquides sont avantageusement introduites dans des parties verticalement opposées de la colonne (7) où elles circulent donc à contre sens l'une de l'autre par un simple effet gravitationnel. L'ouverture permettant l'introduction de la phase plus dense est avantageusement positionnée dans la partie supérieure de la colonne (7) mais en dessous de la zone de décantation que constitue l'extrémité supérieure de la colonne (7). De même l'ouverture permettant l'introduction de la phase la moins dense est avantageusement positionnée dans la partie inférieure de la colonne (7) mais au-dessus de la zone de décantation que constitue l'extrémité inférieure de la colonne (7).

Des moyens d'agitation tels que précédemment décrits sont avantageusement compris dans le réacteur (7) pour permettre le mélange intime des deux phases liquides.

L'eau saline à traiter (1) est avantageusement de l'eau de mer et est introduite, par exemple au moyen d'une pompe vers la colonne (7) où les ions dissous dans l'eau sont transférés totalement ou partiellement vers la phase organique à savoir, dans ce cas particulier, du Calix[4]Est, dissous à hauteur de 0,3M dans du MSA 6. La phase organique non miscible à l'eau, contient donc des molécules solvatantes d'ions, à forte affinité pour au moins certains des ions à transférer. Pour cet exemple particulier où la phase organique non chargée en ion (10) arrivant en tête de colonne est plus dense que l'eau à traiter (1), la phase organique non chargée (10) descend dans la colonne en se chargeant en ions extraits de l'eau saline à traiter (1) pour atteindre l'extrémité inférieure de la colonne (7) où elle s'accumule par décantation après coalescence. A l'inverse, l'eau à traiter (1) injectée dans la partie inférieure de la colonne (7) remonte par densité différentielle (principe d'Archimède) tout en cédant petit à petit ses ions à la phase organique descendante, pour atteindre l'extrémité supérieure de la colonne (7) en tant qu'eau traitée (2) ou dessalée. Cette eau traitée est dessalée et/ou déionisée en tout ou partie, c'est-à-dire qu'elle a perdu tout ou au moins une partie des sels, et/ou des ions constituants ces sels, qu'elle comprenait avant son passage dans le réacteur (7). Par exemple elle est déchlorée ou décarbonatée.

### PROCÉDÉ : CHAUFFAGE DE LA PHASE ORGANIQUE

La phase organique chargée en ions (11) est alors pompée vers un premier échangeur de chaleur (14) pour être réchauffée à une température suffisante (Cf Figure 5) pour permettre de décharger la phase organique chargée (11) des ions extraits de l'eau à traiter (1) à l'étape précédente dans le réacteur (7). La phase organique chargée et chauffée (12) est alors introduite dans la partie supérieure du second réacteur (9) pour être mise en contact avec de l'eau traitée chaude (4).

### SECOND RÉACTEUR

Comme précédemment décrit lorsque le réacteur est une colonne, comme dans cet exemple, les deux phases liquides sont avantageusement introduites dans des parties verticalement opposées de la colonne (9) où elles circulent donc à contre sens l'une de l'autre par un simple effet gravitationnel. L'ouverture permettant l'introduction de la phase plus dense est avantageusement positionnée dans la partie supérieure de la colonne (9) mais en dessous de la zone de décantation que constitue l'extrémité supérieure de cette colonne (9). De même l'ouverture permettant l'introduction de la phase la moins dense est avantageusement positionnée dans la partie inférieure de la colonne (9) mais au-dessus de la zone de décantation que constitue l'extrémité inférieure de cette colonne (9).

Des moyens d'agitation tels que précédemment décrits sont avantageusement compris dans le réacteur (9) pour permettre le mélange intime des deux phases liquides.

### PROCEDE : RECYCLAGE DE LA PHASE ORGANIQUE

L'eau traitée chaude (4) provient avantageusement de l'eau traitée (2) obtenue à l'issue de son traitement dans le réacteur (7) et dont une partie est dirigée par le conduit (3) vers un second échangeur de chaleur (8) pour y être chauffée. L'autre partie de l'eau traitée (15) peut être utilisée.

Cette eau traitée chaude liquide (4) est donc injectée dans la partie inférieure de la colonne (9) et mélangée avec la phase organique liquide chargée et chaude (12). Cette eau chaude liquide (4) remonte par densité différentielle (principe d'Archimède) tout en se chargeant petit à petit du fait de la température de la phase organique descendante, pour atteindre l'extrémité supérieure de la colonne (9) en tant qu'eau chargée en ions (5). Cette eau chargée en ions (5) présente, de préférence, une concentration en ions supérieure à celle présente dans l'eau à traiter (1) et est alors appelée Saumure (ou concentrât). Cette saumure ou concentrât (5) est évacuée après décantation et est dirigée vers l'échangeur de chaleur (8) pour y être refroidie en tant que saumure (6). La phase organique liquide chargée (12) arrivant en tête de colonne (9) est plus dense que l'eau de régénération chaude (4), la phase organique chargée et chaude (12) descend dans la colonne (9) en se déchargeant en ions extraits dans l'eau traitée chaude liquide (4) pour atteindre l'extrémité inférieure de la colonne (9) où elle s'accumule par décantation après coalescence. Cette phase organique régénérée (13) ayant rendue à l'eau de régénération chaude (4) les sels (ou ions) extraits en colonne (9) est alors refroidie par passage à travers l'échangeur de chaleur (14) pour être redirigée (par exemple au moyen d'une pompe) vers la partie supérieure du premier réacteur (7) pour y être introduite et ainsi recyclée en tant que phase organique non chargée (10).

### PHASE ORGANIQUE ET TEMPERATURE DE FONCTIONEMENT

Dans le procédé selon l'invention le contrôle de la température du milieu des premiers et seconds réacteurs (7) et (9) est un facteur important pour en assurer un fonctionnement optimisé. Aussi des moyens de contrôle de la température sont avantageusement inclus dans le dispositif pour permettre de contrôler et éventuellement de modifier la température de ceux-ci. Ceux-ci peuvent comprendre des moyens de mesure de la température (tels que des thermomètres) et/ou des moyens de chauffage (par exemple une source de chaleur) ou de refroidissement (par exemple un refroidisseur).

Dans l'exemple particulier décrit à la Figure 4, de tels moyens peuvent avantageusement être disposés dans ou faire partie de :
1 - un conduit d'arrivée de l'eau à traiter (1) vers le premier réacteur (7),
2 - un conduit amenant la phase organique chargée en ions chaude (12) de l'échangeur (14) vers le second réacteur (9),
3 - un conduit amenant l'eau de régénération chaude (4) de l'échangeur de chaleur (8) vers le second réacteur (9), et/ou
4 - un conduit amenant la phase organique régénérée (10) de l'échangeur de chaleur (14) vers le premier réacteur (7).

Dans le deuxième ou troisième cas mentionné ci-dessus, les moyens de contrôle comprennent avantageusement des moyens de chauffage. Dans le quatrième des cas mentionnés ci-dessus les moyens de contrôle peuvent avantageusement comprendre des moyens de refroidissement.

Ainsi, le procédé selon l'invention permet d'obtenir une saumure plus concentrée en sels (ions) que l'eau à traiter du fait des propriétés intrinsèques d'extraction/d'absorption en ions de la phase organique non miscible à l'eau, qui évoluent en fonction de la température opératoire considérée.

### EXEMPLE 8

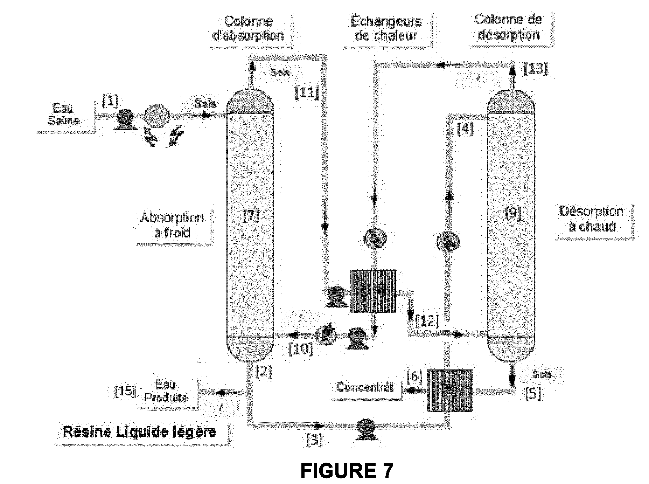

Une variante du dispositif et du procédé décrit à l'exemple 7 est représenté à la figure 7. Dans cette variante la phase organique non miscible à l'eau est moins dense que l'eau à traiter et que la saumure produite. La figure 7 reprend la même numérotation que celle utilisée à la Figure 4. Dans cette variante les colonnes fonctionnent en flux inversés ("la tête en bas"). On y retrouve une section froide à gauche des échangeurs de chaleur et une section chaude à droite des échangeurs de chaleur (8) et (14). Les éléments de ce dispositif sont donc tels que décrits en référence à la Figure 4 et à l'exemple 7.

### EXEMPLE 9

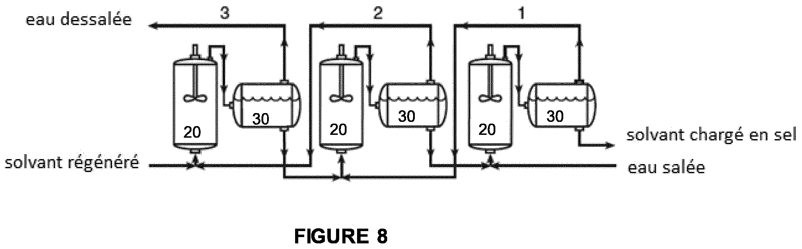

Une autre variante du dispositif est représentée partiellement à la Figure 8. Dans ce dispositif chacune des colonnes (7) et (9) est remplacée par l'association d'un mélangeur rotor/stator (20) à hélice et d'un réservoir à décantation (30). Chacune de ces associations forme une unité d'extraction/de désextraction, qui peut être connectée en série pour pouvoir réaliser une succession d'étape d'absorption ou de régénération. Le nombre d'étapes nécessaires pour effectuer le dessalement d'eau de mer et obtenir une eau où plus de 99% du sodium aura été extrait, sera généralement d'au moins 3, de préférence de 4 ou de 5 étages.

### EXEMPLE 10 : Synthèse des composés MSAC7, MSAC9, MSAC11 et MSAC13

### Schéma de synthèse

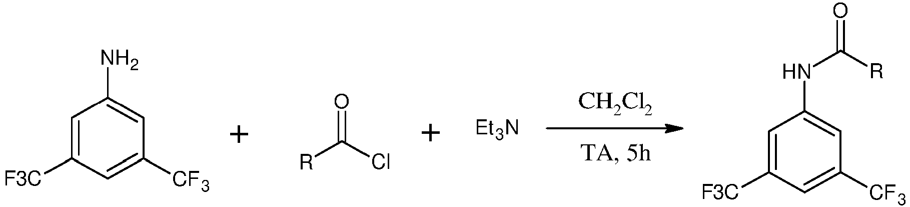

R = n-C7H15 (MSAC7), n-C9H19 (MSAC9), n-C11H23 (MSAC11), n-C13H27 (MSAC13).

### Protocole

A une solution de 3,5-bis(trifluoromethyl)aniline (8.79 mL, 56.29 mmol, 1.0 éq.), de dichlorométhane (40 mL) et de triéthylamine (8.63 mL, 61.92 mmol, 1.1 éq.) est additionnée sous agitation et goutte à goutte le chlorure d'acide (56.29 mmol, 1.0 éq.). La température est contrôlée lors de l'ajout et ne doit pas dépasser 38°C (Point ébullition du dichlorométhane). Le mélange réactionnel est agité pendant 5h à température ambiante. Une solution d'HCl 1M (50 mL) est ajoutée puis la phase organique est lavée. Les lavages successifs sont réalisés par une solution d'HCl 1M (50 mL) et une solution de NaCl saturée (50 mL). La phase organique est séchée sur Na2SO4, filtrée puis le solvant est évaporé sous pression réduite. Le résidu solide est ensuite repris à l'éther de pétrole (froid ou à température ambiante), lavé, filtré puis séché sous vide pour donner l'amide désiré. L'éther de pétrole utilisé est un mélange d'hydrocarbures composé principalement de n-pentane, 2-méthyl pentane et de numéro CAS 64742-49-0 provenant de la société VWR où il est commercialisé sous la dénomination Ether de Pétrole 40-60°C GPR RECTAPUR. Les composés obtenus présentent les caractéristiques suivantes :

| R | Composé | Masse molaire (g/mole) | T° éther pétrole | Rendement | Aspect | Point de fusion |
|---|---|---|---|---|---|---|
| n-C7H 15 | MSAC7 | 355,3 | Froid (-20°C) | 91% | Solide blanc | 43-44°C |
| n-C9H19 | MSAC9 | 383,3 | Ambiante | 92% | Solide blanc | 79-81°C |
| n-C11H23 | MSAC11 | 411,4 | Ambiante | 92% | Solide blanc | 60-61°C |
| n-C13H27 | MSAC13 | 439,5 | Ambiante | 90% | Solide blanc | 53-54°C |

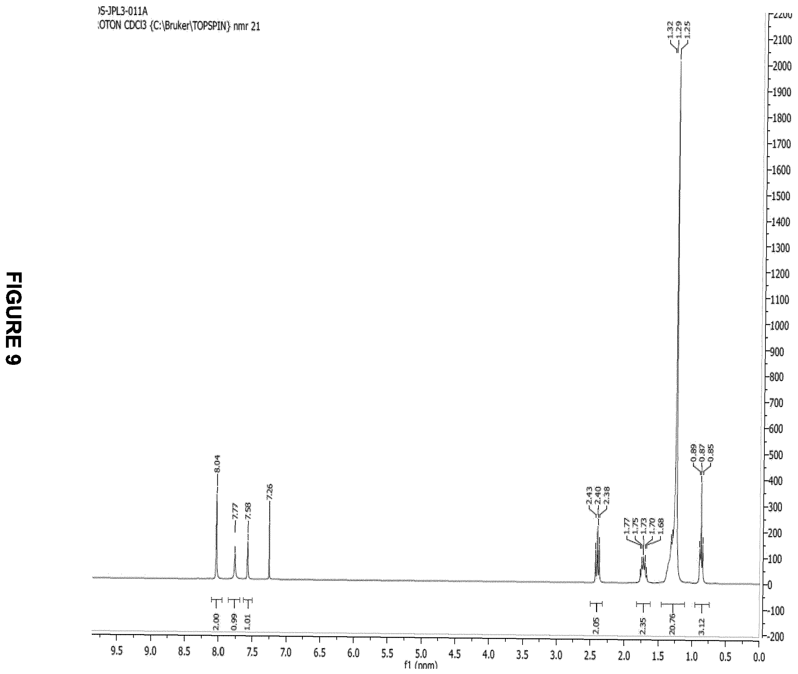

Les composés MSAC7, MSAC9, MSAC11 et MSAC13 ont pour dénominations IUPAC respectives : N-[3,5-bis(trifluoromethyl)phenyl]octanamide, N-[3,5-bis(trifluoromethyl)phenyl]decanamide, N-[3,5-bis(trifluoromethyl)phenyl]dodecanamide, N-[3,5-bis(trifluoromethyl)phenyl]tetradecanamide et ont de plus été identifiés par spectrométrie RMN. La figure 9, représente le spectre RMN (CDCl3, 300 MHz) du composé MSAC11 dont les pics sont les suivants : 1H RMN (CDCl3, 300 MHz) : δ (ppm) = 0.87 (t, 3J = 7.0 Hz, 3H), 1.20-1.35 (m, 20H), 1.73 (quint., 3J = 7.0 Hz, 2H), 2.40 (t, , 3J = 7.0 Hz, 2H), 7.58 (s, 1H), 7.77 (bs, 1H), 8.04 (s, 2H).

### EXEMPLE 11 : Extraction de chlorure de sodium d'une solution aqueuse par des formulations comprenant une MSA de la famille des amides et la MEC Calix[4]Est en présence d'un fluidifiant (Chloroforme CHCl₃) et comparaison avec d'autres MSA et composés.

Les MSA de la famille des amides utilisés sont les composés MSAC7, MSAC9, MSAC11, et MSAC13 dont la synthèse est décrite à l'exemple 10. A titre comparatif la MSA3 et la 3,5-Di(trifluoromethyl)aniline (No. CAS 328-74-5) ont également été utilisées dans la préparation de compositions extractantes.

La composition d'extraction est obtenue en solubilisant une quantité de 4-tert-butyl Calix[4]arène tétraethyl Ester (CAS n°97600-39-0) et de MSA dans du chloroforme CHCl₃ pour obtenir une concentration finale après solubilisation des MEC et MSA de respectivement 0.3 mol/L de Calix[4]Est et de 0,3 mol/L de MSA. Ces formulations scellées ont ensuite été mises en agitation orbitalaire à 500 tours/minute pendant 2 heures après avoir ajouté un volume équivalent d'eau distillée deux fois afin de permettre une saturation à l'eau de l'ensemble de la formulation et un contrôle de PH en sortie (PH=7). La composition d'extraction est alors mise au repos pour décantation. Toutes les compositions testées sont stables et se décantent rapidement (quelques minutes au plus).

Une solution aqueuse de NaCl à 0,4 mol/L est préparée à partir d'une eau distillée deux fois.

La composition organique d'extraction est alors légèrement chauffée pour favoriser la solubilisation des composés par pistolet à air chaud (température d'environ 50 à 60°C) pendant quelques secondes (10 à 30 secondes) jusqu'à obtenir une solution limpide.

3 mL de la composition organique d'extraction sont alors prélevés en phase inférieure du mélange diphasique décanté et transvasés vers un flacon contenant 3 mL de l'eau salée à 0,4 M de NaCl, puis le flacon est scellé et mis sous agitation orbitalaire (à 500 tours par minutes), pendant 2 heures à température ambiante (TA) c'est-à-dire entre 20 et 25°C. Pour le cas de l'extraction à 60°C une agitation magnétique (à 500 tours par minutes), pendant 2 heures, est réalisée avec chauffage indirect dans un moule métallique sur une plaque chauffante. Il est vérifié que des gouttelettes de l'ordre de 1-2 mm sont bien présentes au cours de ces agitations pour être certain d'atteindre un équilibre dans la répartition du NaCl entre les deux phases liquides en fin d'agitation. L'aspect des phases organiques et aqueuses est limpide et incolore ou légèrement trouble.

Une fois les 2 heures d'agitation réalisées, l'agitation est arrêtée et l'ensemble est mis au repos pour décantation pendant environ 10 minutes, tout au moins jusqu'à séparation totale des deux phases, à la température de l'essai. Puis, la phase aqueuse supérieure est prélevée puis agitée et diluée pour analyse de sa salinité par une Chromatographie Ionique de marque Metrohm intégrant une colonne d'analyse des cations, adaptée et une colonne d'analyse des anions, adaptée. De même, la solution aqueuse initiale de NaCl à 0,4M est également analysée par cette chromatographie ionique pour déterminer sa concentration relative molaire en sodium et en chlorures. Toutes les extractions et analyses ont étés dupliquées. Le tableau ci-dessous présente les résultats observés pour une répartition iso-molaire entre MSA et MEC :

| **MSA testée** | **Concent ration de MEC en Mol/L** | **Concen tration de MSA en Mol/L** | **Aspect après saturation à l'eau** | **T (°C) d'extra ction** | **% en moles de Na+ extrait de l'eau à traiter** | **% en moles de CI- extrait de l'eau à traiter** |
|---|---|---|---|---|---|---|
| MSAC13 | 0,300 | 0,300 | Légèrement trouble | TA | 22,7% | 26,2% |
| MSAC13 | 0,300 | 0,300 | Légèrement trouble | TA | 23,8% | 27,4% |
| MSAC13 | 0,300 | 0,300 | Légèrement trouble | 60°C | 5,9% | 6,9% |
| MSAC13 | 0,300 | 0,300 | Légèrement trouble | 60°C | 9,0% | 9,4% |
| MSAC11 | 0,300 | 0,300 | Légèrement trouble, qq cristaux | TA | 22,4% | 24,5% |
| MSAC11 | 0,300 | 0,300 | Légèrement trouble, qq cristaux | TA | 23,1% | 25,3% |
| MSAC11 | 0,300 | 0,300 | Légèrement trouble, qq cristaux | 60°C | 3,4% | 4,9% |
| MSAC11 | 0,300 | 0,300 | Légèrement trouble, qq cristaux | 60°C | 7,1% | 7,7% |
| MSAC9 | 0,300 | 0,300 | Légèrement trouble | TA | 25,2% | 27,1% |
| MSAC9 | 0,300 | 0,300 | Légèrement trouble | TA | 21,7% | 23,8% |
| MSAC9 | 0,300 | 0,300 | Légèrement trouble | 60°C | 5,4% | 6,9% |
| MSAC9 | 0,300 | 0,300 | Légèrement trouble | 60°C | 5,7% | 8,0% |
| MSAC7 | 0,304 | 0,304 | Limpide | TA | 23,6% | 26,3% |
| MSAC7 | 0,304 | 0,304 | Limpide | TA | 22,8% | 25,9% |
| MSAC7 | 0,304 | 0,304 | Limpide | 60°C | 5,5% | 7,6% |
| MSAC7 | 0,304 | 0,304 | Limpide | 60°C | 5,4% | 7,3% |
| MSA3 (Référenc e) | 0,300 | 0,300 | Limpide | TA | 6,8% | 8,2% |
| MSA3 (Référenc e) | 0,300 | 0,300 | Limpide | TA | 7,6% | 8,8% |
| MSA3 (Référenc e) | 0,300 | 0,300 | Limpide | 60°C | non détecté | non détecté |
| MSA3 (Référenc e) | 0,300 | 0,300 | Limpide | 60°C | non détecté | non détecté |
| AnilineF* | 0,300 | 0,295 | Limpide | TA | 2,0% | 4,3% |
| AnilineF* | 0,300 | 0,295 | Limpide | TA | 1,8% | 2,9% |
| AnilineF* | 0,300 | 0,295 | Limpide | 60°C | non détecté | non détecté |
| AnilineF* | 0,300 | 0,295 | Limpide | 60°C | non détecté | non détecté |

| | | | | | | |
|---|---|---|---|---|---|---|
| *3,5-Di(trifluoromethyl)aniline ( | | | | | | |

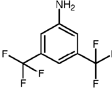

)

Les résultats moyens pour l'extraction molaire du chlorure de sodium peuvent donc être résumés dans le tableau suivant :

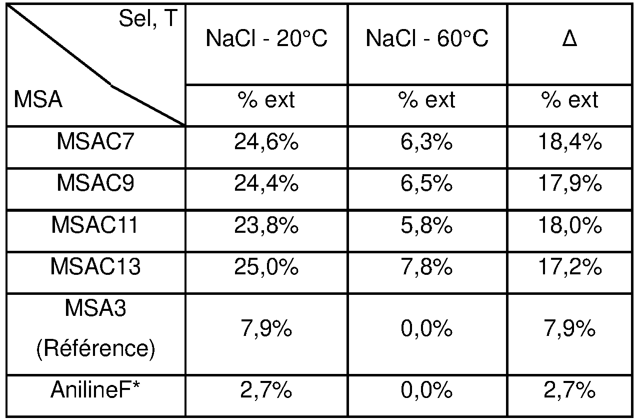

Il apparait donc d'une part que les solvatants anioniques de la famille des amides (MSAC7 à 13) selon l'invention sont plus actifs que les solvatants anioniques de la famille des alcools (MSA3). En particulier ils permettent une capture efficace à température ambiante et un relargage suffisant des espèces ioniques à une température plus élevée mais suffisamment basse (notamment inférieure à 150°C). Il apparait également que la version aminée AnilineF* est encore moins active que l'alcool à concentration identique. Cependant ces composés peuvent être utilisés dans des compositions extractantes selon l'invention simplement en augmentant la concentration de ceux-ci au-delà de 2 mol/L de MSA (voir exemples 6).

Cette suractivité des amides est de plus maintenue lorsque la chaine alkyle de la fonction amide est allongée de C₇H₁₅ à C₁₃H₂₇, ce qui permet de s'assurer d'une bonne insolubilité à l'eau de cette famille de solvatants anioniques.

### Exemple 12 : Extraction de chlorure de sodium d'une solution aqueuse par des formulations comprenant la MSAC7 de la famille des amides à quatre concentrations différentes et la MEC Calix[4]Est à concentration constante en présence d'un fluidifiant (CHCl₃) et comparaison des performances d'extraction associées.

Quatre compositions d'extraction ont été obtenues en solubilisant une quantité constante de 4-tert-butyl Calix[4]arene tétraethyl Ester (CAS η°97600-39-0) et quatre quantités croissantes de MSAC7 dans du chloroforme CHCl₃ pour obtenir quatre concentrations finales après solubilisation du MEC et du MSA, de 0.34 à 0,36 mol/L de Calix[4]Est et de respectivement 0,36 mol/L, 0,71 mol/L, 1,09 mol/L et 1,49 mol/L de MSAC7. Ces quatre formulations scellées ont ensuite été mises en agitation orbitalaire à 500 tours/minute pendant 2 heures après avoir ajouté un volume équivalent d'eau distillée deux fois afin de permettre une saturation à l'eau de l'ensemble de la formulation et un contrôle de PH en sortie (PH=7). La composition d'extraction est alors mise au repos pour décantation. Toutes les compositions testées sont stables et se décantent rapidement (quelques minutes au plus).

Une solution aqueuse de NaCl à 0,3 mol/L est préparée à partir d'une eau distillée deux fois.

3 mL de chaque composition organique d'extraction sont alors prélevés en phase inférieure du mélange diphasique décanté et transvasés vers quatre flacons contenant chacun 3 mL de l'eau salée à 0,3 M de NaCl, puis les flacons sont scellés et mis sous agitation orbitalaire (à 500 tours par minutes), pendant 2 heures à température ambiante (TA) c'est-à-dire entre 20 et 25°C. Pour le cas de l'extraction à 60°C une agitation magnétique (à 500 tours par minutes), pendant 2 heures, est réalisé avec chauffage indirect dans un moule métallique sur une plaque chauffante. Il est vérifié que des gouttelettes de l'ordre de 1-2 mm sont bien présentes au cours de ces agitations pour être certain d'atteindre un équilibre dans la répartition du NaCl entre les deux phases liquides en fin d'agitation. L'aspect des phases organiques et aqueuses est limpide et incolore pour ces 4 formulations testées.

Une fois les 2 heures d'agitation réalisées, l'agitation est arrêtée et l'ensemble est mis au repos pour décantation pendant environ 10 minutes, tout au moins jusqu'à séparation totale des deux phases, à la température de l'essai. Puis, les quatre phases aqueuses supérieures sont prélevées séparément puis agitées et diluées pour analyse de leur salinité par une Chromatographie Ionique de marque Metrohm intégrant une colonne d'analyse des cations, adaptée et une colonne d'analyse des anions, adaptée. De même, la solution aqueuse initiale de NaCl à 0,3M est également analysée par cette chromatographie ionique pour déterminer sa concentration relative molaire en sodium et en chlorures. Toutes les extractions et analyses ont étés dupliquées. Le tableau ci-dessous présente les résultats observés pour quatre répartitions molaires entre MSA et MEC:

| Concen tration de MEC en Mol/L | Concen tration de MSAC7 en Mol/L | Ratios de concentration [MSAC7]/ [MSA] | Aspect après saturation à l'eau | T (°C) d'extraction | % molaire de Na+ extrait de l'eau à traiter | % molaire de Cl-extrait de l'eau à traiter |
|---|---|---|---|---|---|---|
| 0,345 | 0,358 | 1,04 | Limpide | TA | 27,9% | 27,9% |
| 0,345 | 0,358 | 1,04 | Limpide | TA | 21,7% | 27,2% |
| 0,345 | 0,358 | 1,04 | Limpide | 60°C | 9,9% | 13,0% |
| 0,345 | 0,358 | 1,04 | Limpide | 60°C | 13,1% | 14,1% |
| 0,341 | 0,708 | 2,08 | Limpide | TA | 44,2% | 44,4% |
| 0,341 | 0,708 | 2,08 | Limpide | TA | 45,1% | 44,4% |
| 0,341 | 0,708 | 2,08 | Limpide | 60°C | 28,1% | 26,6% |
| 0,341 | 0,708 | 2,08 | Limpide | 60°C | 28,2% | 27,4% |
| 0,349 | 1,088 | 3,12 | Limpide | TA | 60,0% | 51,2% |
| 0,349 | 1,088 | 3,12 | Limpide | TA | 59,8% | 52,9% |
| 0,349 | 1,088 | 3,12 | Limpide | 60°C | 37,7% | 31,0% |
| 0,349 | 1,088 | 3,12 | Limpide | 60°C | 39,5% | 30,4% |
| 0,358 | 1,488 | 4,16 | Limpide | TA | 71,2% | 64,6% |
| 0,358 | 1,488 | 4,16 | Limpide | TA | 65,0% | 65,6% |
| 0,358 | 1,488 | 4,16 | Limpide | 60°C | 48,4% | 45,6% |
| 0,358 | 1,488 | 4,16 | Limpide | 60°C | 46,9% | 39,9% |

Les résultats moyens pour l'extraction du chlorure de sodium peuvent donc être résumés dans le tableau suivant :

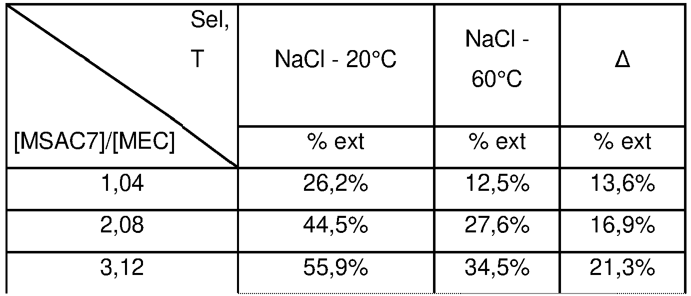

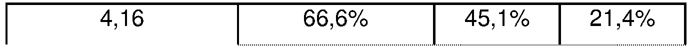

Il apparait une hausse régulière et quasiment linéaire du taux d'extraction du NaCl avec une augmentation de la concentration relative en MSAC7, que ce soit à température ambiante ou à 60°C, montrant l'importance d'une poly-solvatation de l'anion Chlorure par le MSA pour permettre une bonne extraction du NaCl. Notons aussi que tous les MEC ne sont pas employés à 0,3 M de salinité initiale en NaCl, laissant de la place à une extraction supplémentaire à plus forte salinité.

L'invention n'est pas limitée aux modes de réalisation présentés et d'autres modes de réalisation apparaîtront clairement à l'homme du métier. Il est notamment possible d'employer ce procédé pour valoriser l'eau de nombre de sources d'eaux naturelles ou industrielles salines. Il est également possible d'employer ce procédé afin de faire de la reconcentration de sels en augmentant la température de régénération ou pour extraire sélectivement certains sels ayant par exemple une certaine valeur économique ou entartrants. De plus, moyennant certains aménagements, ce procédé pourra traiter des eaux de production pétrolière ou des eaux industrielles pour la production d'eau de procédé, pour limiter les impacts environnementaux associés aux rejets d'eau salines en milieux naturels.

L'invention pourra aussi intégrer des modes de réalisation ou plusieurs MEC seront dissous dans un MSA, un mélange de MSA ou un MSA et un fluidifiant ou un mélange de MSA et de fluidifiants afin de permettre l'extraction d'un plus large panel de cations et d'anions ; leurs contre-ions associés.

## Revendications

1. - Procédé de traitement d'une eau saline par déionisation thermique comprenant l'extraction d'au moins deux espèces ioniques, lesdites espèces ioniques comprenant une espèce anionique et une espèce cationique et étant présentes dans de l'eau à traiter, ledit procédé comprenant les étapes suivantes :
a) le mélange dans un premier réacteur, à une première température, entre une phase organique hydrophobe liquide et l'eau saline à traiter, ladite eau à traiter étant à l'état liquide, pour l'obtention subséquente d'une eau traitée liquide dessalée et/ou déionisée en tout ou partie et d'une phase organique liquide hydrophobe chargée en lesdites espèces ioniques, ladite phase hydrophobe comprenant :
- au moins un premier composé organique solvatant les anions, protique et hydrophobe dont le pKa dans l'eau à 25°C est d'au moins 9, de préférence 10,5 et est préférentiellement inférieur au pKa de l'eau à 25°C, ou du moins inférieur à 15 à 25°C et dont la solubilité dans l'eau à 25°C est inférieure à 0,01 mol/l, ledit premier composé étant un composé représenté par la Formule (B) : 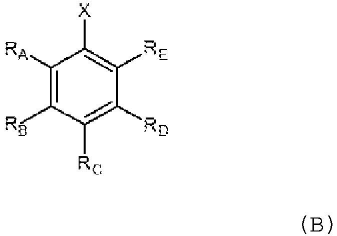
dans laquelle au moins un quelconque des radicaux R_{A}, R_{B}, R_{C}, R_{D} et R_{E} identiques ou différents, est un atome halogène ou un groupement électro-attracteur du groupe suivant : F, Cl, Br ; CₘF₂ₘ₊₁ avec m ≤ 4, où m est un entier non nul ; CF₂CF₂CₚH₂ₚ₊₁ avec p ≤ 4, où p est un entier ; CF₂CₚH₂ₚ₊₁ avec p ≤ 4, où p est un entier ; CH₂CₚF₂ₚ₊₁ avec p ≤ 4, où p est un entier ; OCH_{2C}F₃ ; C(=O)CF₃ ; CₘHₙFₚCl_{q}Brₛ avec m ≤ 4, où n, p, q, s sont des entiers dont au moins p, q ou s est non nul ; C(=O)OCₘH₂ₘ₊₁ avec m ≤ 4, où m est un entier ; et C(=O)CₘH₂ₘ₊₁ avec m ≤ 4, où m est un entier,
le ou les radicaux R_{A}, R_{B}, R_{C}, R_{D} et R_{E} restants sont choisis, identiques ou différents, parmi les radicaux non électro-attracteurs suivants : H ; CH₃ ; CH₂CH₃ ; CH₂CH₂CₚF₂ₚ₊₁ avec p ≤ 4, ou p est un entier ; CₘH₂ₘ₋₁ avec m ≤ 10, où m est un entier non nul ; et CₘH₂ₘ₊₁ avec m ≤ 10, où m est un entier non nul ; où un seul des radicaux R_{A} à R_{E} peut être un de ces deux derniers radicaux CₘH₂ₘ₋₁ et CₘH₂ₘ₊₁ ;
et dans laquelle X est choisi parmi les radicaux suivants : OH ; 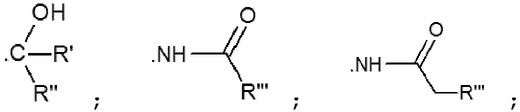 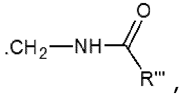
où R' et R'', identiques ou différents, sont choisis parmi les radicaux suivants : CₙH₂ₙ₋₁ avec n ≤ 4, où n est un entier non nul ; CₙH₂ₙ₊₁ avec n ≤ 4, où n est un entier non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; CₘHₙFₚCl_{q}Brₛ avec m ≤ 4, où n, p, q, s sont des entiers dont au moins p, q ou s est non nul ; et un radical aryle de formule (b) : 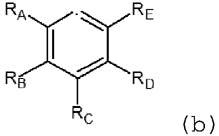
où R_{A}, R_{B}, R_{C}, R_{D} et R_{E}, identiques ou différents, sont tels que précédemment définis dans la formule (B) ;
et où R‴ est choisi parmi les radicaux suivants : CₘH₂ₘ₊₁ avec m ≤ 20, où m est un entier ; CₘH₂ₘ₋₁ avec m ≤ 20, où m est un entier non nul ; CₘHₙFₚCl_{q}Brₛ avec m ≤ 10, où n, p, q, s sont des entiers dont au moins p, q ou s est non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; et un radical aryle de formule (b) : 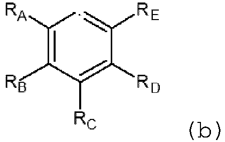
où R_{A}, R_{B}, R_{C}, R_{D} et R_{E}, identiques ou différents, sont tels que précédemment définis dans la formule (B) ; et,
- au moins un deuxième composé organique extractant les cations et hydrophobe choisi parmi les éther-couronnes, les cryptands ou les calixarènes fonctionnalisés et présentant une constante de complexation de ladite espèce cationique dont la valeur Log K, dans le méthanol à 25 °C, est supérieure à 3 et inférieure à 9 ;
b) la séparation, d'une part, de ladite eau traitée liquide dessalée et/ou déionisée en tout ou partie et d'autre part de ladite phase organique liquide chargée en lesdites espèces ioniques ; et
c) le mélange, à une deuxième température plus élevée, en phase liquide, dans un deuxième réacteur de ladite phase organique liquide chargée en lesdites espèces ioniques et d'eau de régénération liquide, pour l'obtention subséquente d'une phase organique liquide régénérée et d'eau liquide de régénération chargée en lesdites espèces ioniques, la différence entre lesdites première et deuxième températures allant de 30 °C à 150 °C, la deuxième température étant plus élevée que la première température.

2. - Procédé selon la revendication 1, **caractérisé par le fait que** le procédé ne comprend pas d'étape où le pH de l'eau de régénération liquide est significativement modifié, c'est-à-dire au-delà d'une variation de pH de +/-2, par exemple +/- 1 par rapport à l'eau à traiter.

3. - Procédé selon l'une des revendications 1 et 2, **caractérisé par le fait que** ledit procédé comprend les étapes subséquentes de :
d) séparation de ladite phase organique liquide régénérée et de l'eau liquide de régénération chargée en lesdites espèces ioniques ;
e) mise en contact thermique indirecte de ladite phase organique liquide chargée en lesdites espèces ioniques et de ladite phase organique liquide régénérée.

4. - Procédé selon l'une quelconque des revendications 1 à 3, **caractérisé par le fait que** le procédé comprend une étape de chauffage de l'eau de régénération liquide effectuée avant l'étape c).

5. - Procédé selon l'une quelconque des revendications 1 à 4, **caractérisé par le fait que** l'espèce anionique est du chlorure ou du sulfate.

6. - Procédé selon l'une des revendications 1 à 5, **caractérisé par le fait que** le composé (B) est un composé dans lequel X représente :

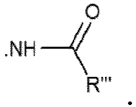

7. - Procédé selon la revendication 6, **caractérisé par le fait que** le composé (B) est représenté par la formule : 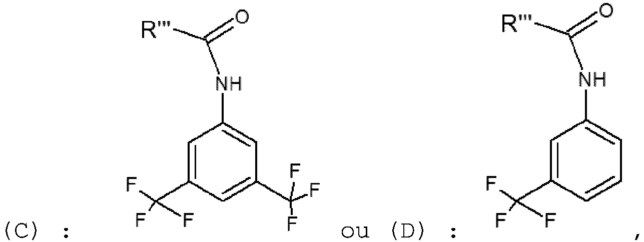
dans laquelle R''' est choisi parmi les radicaux suivants : CₘH₂ₘ₊₁ avec m ≤ 20, de préférence ≤ 15 où m est un entier ; CₘH₂ₘ₋₁ avec m ≤ 20, où m est un entier non nul ; CₘHₙFₚCl_{q}Brₛ avec m ≤ 10, où n, p, q, s sont des entiers dont au moins p, q ou s est non nul ; et un radical aryle de formule (b) : 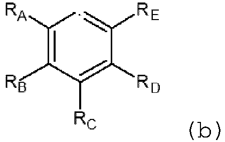
dans laquelle au moins un quelconque des radicaux R_{A}, R_{B}, R_{C}, R_{D} et R_{E}, identiques ou différents, est un atome halogène ou un groupement électro-attracteur, en particulier un radical halogéné, du groupe suivant : F, Cl, Br ; CₘF₂ₘ₊₁ avec m ≤ 4, où m est un entier non nul ; CF₂CF₂CₚH₂ₚ₊₁ avec p ≤ 4, où p est un entier ; CF₂CₚH₂ₚ₊₁ avec p ≤ 4, où p est un entier ; CH₂CₚF₂ₚ₊₁ avec p ≤ 4, où p est un entier ; OCH_{2C}F₃ ; C(=O)CF₃ ; CₘHₙFₚCl_{q}Brₛ avec m ≤ 4, où n, p, q, s sont des entiers dont au moins p, q ou s est non nul ; C(=O)OCₘH₂ₘ₊₁ avec m ≤ 4, où m est un entier ; et C(=O)CₘH₂ₘ₊₁ avec m ≤ 4, où m est un entier, le ou les radicaux R_{A}, R_{B}, R_{C}, R_{D} et R_{E} restants sont choisis, identiques ou différents, parmi les radicaux non électro-attracteurs suivants : H ; CH₃ ; CH₂CH₃ ; CH₂CH₂CₚF_{2p + 1} avec p ≤ 4, ou p est un entier ; CₘH₂ₘ₋₁ avec m ≤ 10, où m est un entier non nul ; et CₘH₂ₘ₊₁ avec m ≤ 10, où m est un entier non nul,
où un seul des radicaux R_{A} à R_{E} peut être un de ces deux derniers radicaux CₘH₂ₘ₋₁ et CₘH₂ₘ₊₁.

8. - Procédé selon la revendication 7, **caractérisé par le fait que** le radical R''' est n-C₇H₁₅, n-C₉H₁₉, n-C₁₁H₂₃ ou n-C₁₃H₂₇.

9. - Procédé selon l'une des revendications 1 à 8, **caractérisé par le fait que** le deuxième composé organique est un éther couronne, notamment un éther couronne ayant 14 à 80 atomes de carbone, et peut être choisi dans le groupe constitué du 6,7,9,10,12,13,20,21,23,24-décahydrodibenzo[b,k] [1,4,7,10,12,16,19] heptaoxa-cyclohenicosine (DB21C7), benzo[b]-1,4,7,10,13-pentaoxacyclopentadécane (B15C5), perhydrobenzo[b]-1,4,7,10,13-pentaoxacyclopentadécane (C15C5), dicyclohexano-1,4,7,10,13,16-hexaoxacyclooctadécane (DC18C6), dibenzo[b,k]-1,4,7,10,13,16-hexaoxacyclooctadécane (DB18C6) et 6,7,9,10,12,13,20,21,23,24,26,27-dodécahydrodibenzo[b,n][1,4,7,10,13,16,19,22]octaoxa-cyclotétracosine (DB24C8).

10. - Procédé selon l'une des revendications 1 à 8, **caractérisé par le fait que** le deuxième composé organique est un calixarène substitué pouvant comprendre, par exemple, de 32 à 80 atomes de carbone, par exemple de 50 à 70 atomes de carbone, tel que l'ester tétraéthylique de l'acide 4 -tert-butylcalix[4]-arène-O',O',O",O‴-tétraacétique, tel que le Calix[4]Est.

11. - Procédé selon l'une des revendications 1 à 10, **caractérisé par le fait que** la phase organique hydrophobe liquide utilisée à l'étape a) contient également un fluidifiant.

12. - Procédé selon la revendication 11, **caractérisé par le fait que** le fluidifiant est choisi dans le groupe constitué de solvants aromatiques polaires.

13. - Composition pour la mise en œuvre du procédé selon l'une des revendications 1 à 12, **caractérisé par le fait qu'**elle comprend :
- au moins un premier composé organique solvatant les anions, protique et hydrophobe dont le pKa dans l'eau à 25°C est d'au moins 9, de préférence 10,5 et est préférentiellement inférieur au pKa de l'eau à 25°C, ou du moins inférieur à 15 à 25°C et dont la solubilité dans l'eau à 25°C est inférieure à 0,01 mol/l, ledit premier composé étant un composé représenté par la Formule (B) : 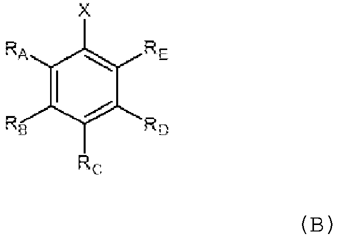
dans laquelle au moins un quelconque des radicaux R_{A}, R_{B}, R_{C}, R_{D} et R_{E} identiques ou différents, est un atome halogène ou un groupement électro-attracteur du groupe suivant : F, Cl, Br ; CₘF₂ₘ₊₁ avec m ≤ 4, où m est un entier non nul ; CF₂CF₂CₚH₂ₚ₊₁ avec p ≤ 4, où p est un entier ; CF₂CₚH₂ₚ₊₁ avec p ≤ 4, où p est un entier ; CH₂CₚF₂ₚ₊₁ avec p ≤ 4, où p est un entier ; OCH_{2C}F₃ ; C(=O)CF₃ ; CₘHₙFₚCl_{q}Brₛ avec m ≤ 4, où n, p, q, s sont des entiers dont au moins p, q ou s est non nul ; C(=O)OCₘH₂ₘ₊₁ avec m ≤ 4, où m est un entier ; et C(=O)CₘH₂ₘ₊₁ avec m ≤ 4, où m est un entier,
le ou les radicaux R_{A}, R_{B}, R_{C}, R_{D} et R_{E} restants sont choisis, identiques ou différents, parmi les radicaux non électro-attracteurs suivants : H ; CH₃ ; CH₂CH₃ ; CH₂CH₂CₚF₂ₚ₊₁ avec p ≤ 4, ou p est un entier ; CₘH₂ₘ₋₁ avec m ≤ 10, où m est un entier non nul ; et CₘH₂ₘ₊₁ avec m ≤ 10, où m est un entier non nul ; où un seul des radicaux R_{A} à R_{E} peut être un de ces deux derniers radicaux CₘH₂ₘ₋₁ et CₘH₂ₘ₊₁ ;
et dans laquelle X est choisi parmi les radicaux suivants : OH ; 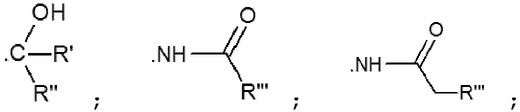 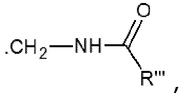
où R' et R'', identiques ou différents, sont choisis parmi les radicaux suivants : CₙH₂ₙ₋₁ avec n ≤ 4, où n est un entier non nul ; CₙH₂ₙ₊₁ avec n ≤ 4, où n est un entier non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; CₘHₙFₚCl_{q}Brₛ avec m ≤ 4, où n, p, q, s sont des entiers dont au moins p, q ou s est non nul ; et un radical aryle de formule (b) : 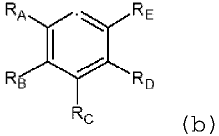
où R_{A}, R_{B}, R_{C}, R_{D} et R_{E}, identiques ou différents, sont tels que précédemment définis dans la formule (B) ;
et où R‴ est choisi parmi les radicaux suivants : CₘH₂ₘ₊₁ avec m ≤ 20, où m est un entier ; CₘH₂ₘ₋₁ avec m ≤ 20, où m est un entier non nul ; CₘHₙFₚCl_{q}Brₛ avec m ≤ 10, où n, p, q, s sont des entiers dont au moins p, q ou s est non nul ; CH₂CH₂CₚF₂ₚ₊₁ avec p ≤ 4, ou p est un entier ; CH₂CₚF₂ₚ₊₁ avec p ≤ 4, ou p est un entier ; CF₂CₚH₂ₚ₊₁ avec p ≤ 4, où p est un entier ; CF₂CF₂CₚH₂ₚ₊₁ avec p ≤ 4, où p est un entier ; CₘF₂ₘ₊₁ avec m ≤ 4, où m est un entier non nul ; et un radical aryle de formule (b) : 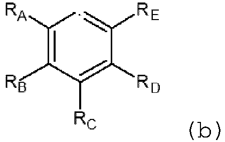
- où R_{A}, R_{B}, R_{C}, R_{D} et R_{E}, identiques ou différents, sont tels que précédemment définis dans la formule (B) ;
- au moins un second composé organique hydrophobe extractant les cations choisi parmi les éther-couronnes, les cryptands ou les calixarènes fonctionnalisés et présentant une constante de complexation de ladite espèce cationique dont la valeur Log K, dans le méthanol à 25 °C, est supérieure à 3 et inférieure à 9.

## Patentansprüche

1. - Verfahren zur Behandlung von salzhaltigem Wasser durch thermische Deionisation, umfassend die Extraktion von mindestens zwei ionischen Spezies, wobei die ionischen Spezies eine anionische Spezies und eine kationische Spezies umfassen und in zu behandelndem Wasser vorhanden sind, wobei das Verfahren die folgenden Schritte umfasst:
a) Mischen in einem ersten Reaktor bei einer ersten Temperatur zwischen einer flüssigen hydrophoben organischen Phase und dem zu behandelnden Salzwasser, wobei das zu behandelnde Wasser in flüssigem Zustand vorliegt, um anschließend ein vollständig oder teilweise entsalztes und/oder entionisiertes flüssiges behandeltes Wasser und eine hydrophobe flüssige organische Phase, die mit den ionischen Spezies beladen ist, zu erhalten, wobei die hydrophobe Phase umfasst:
- mindestens eine erste anionensolvatisierende, protische und hydrophobe organische Verbindung, deren pKa in Wasser bei 25°C mindestens 9, vorzugsweise 10,5 beträgt und vorzugsweise kleiner als der pKa von Wasser bei 25°C ist, oder zumindest kleiner als 15 bei 25°C ist, und deren Löslichkeit in Wasser bei 25°C weniger als 0,01 mol/l beträgt, wobei die erste Verbindung eine Verbindung ist, die durch die Formel (B) dargestellt wird: 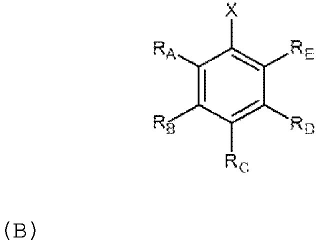
worin mindestens einer der Reste R_{A}, R_{B}, R_{C}, R_{D} und R_{E}, die gleich oder verschieden sind, ein Halogenatom oder eine elektronenanziehende Gruppe der folgenden Gruppe ist: F, Cl, Br; CₘF₂ₘ₊₁ mit m ≤ 4, wobei m eine ganze Zahl ungleich Null ist; CF₂CF₂CₚH₂ₚ₊₁ mit p ≤ 4, wobei p eine ganze Zahl ist; CF₂CₚH₂ₚ₊₁ mit p ≤ 4, wobei p eine ganze Zahl ist; CH₂CₚF₂ₚ₊₁ mit p ≤ 4, wobei p eine ganze Zahl ist; OCH₂CF₃; C(=O)CF₃; CₘHₙFₚCl_{q}Brₛ mit m ≤ 4, wobei n, p, q, s ganze Zahlen sind, von denen mindestens p, q oder s nicht Null ist; C(=O)OCₘH₂ₘ₊₁ mit m ≤ 4, wobei m eine ganze Zahl ist; und C(=O)CₘH₂ₘ₊₁ mit m ≤ 4, wobei m eine ganze Zahl ist,
der oder die verbleibenden Reste R_{A}, R_{B}, R_{C}, R_{D} und R_{E} gleich oder verschieden aus den folgenden nichtelektronenanziehenden Resten ausgewählt sind: H; CH₃; CH₂CH₃; CH₂CH₂CₚF₂ₚ₊₁ mit p ≤ 4, wobei p ist eine ganze Zahl; CₘH₂ₘ₋₁ mit m ≤ 10, wobei m eine ganze Zahl ungleich Null ist; und CₘH₂ₘ₊₁ mit m ≤ 10, wobei m eine ganze Zahl ungleich Null ist; wobei nur einer der Reste R_{A} bis R_{E} einer der beiden letztgenannten Reste CₘH₂ₘ₋₁ und CₘH₂ₘ₊₁ sein kann ;
und worin X aus den folgenden Radikalen ausgewählt ist: OH; 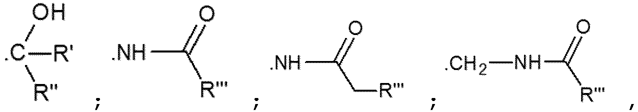
wobei R' und R", die gleich oder verschieden sind, aus den folgenden Resten ausgewählt sind: CₙH₂ₙ₋₁ mit n ≤ 4, wobei n eine ganze Zahl ungleich Null ist; CₙH₂ₙ₊₁ mit n ≤ 4, wobei n eine ganze Zahl ungleich Null ist; CH₂CH₂CₚF₂ₚ₊₁ mit p ≤ 4, wobei p ist eine ganze Zahl; CH₂CₚF₂ₚ₊₁ mit p ≤ 4, wobei p ist eine ganze Zahl; CF₂CₚH₂ₚ₊₁ mit p ≤ 4, wobei p eine ganze Zahl ist ; CF₂CF₂CₚH₂ₚ₊₁ mit p ≤ 4, wobei p eine ganze Zahl ist;
CₘF₂ₘ₊₁ mit m ≤ 4, wobei m eine ganze Zahl ungleich Null ist; CₘHₙFₚCl_{q}Brₛ mit m ≤ 4, wobei n, p, q, s ganze Zahlen sind, von denen mindestens p, q oder s ungleich Null ist; und ein Arylradikal der Formel (b) : 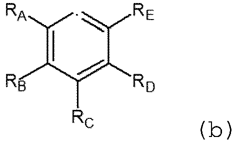
worin R_{A}, R_{C}, R_{C}, R_{D} und R_{E}, die gleich oder verschieden sind, wie zuvor in Formel (B) definiert sind;
und wobei R‴ aus den folgenden Radikalen ausgewählt ist: CₘH₂ₘ₊₁ mit m ≤ 20, wobei m eine ganze Zahl ist; CₘH₂ₘ₋₁ mit m ≤ 20, wobei m eine ganze Zahl ungleich Null ist; CₘHₙFₚCl_{q}Brₛ mit m ≤ 10, wobei n, p, q, s ganze Zahlen sind, von denen mindestens p, q oder s ungleich Null ist; CH₂CH₂CₚF₂ₚ₊₁ mit p ≤ 4, wobei p eine ganze Zahl ist; CH₂CₚF₂ₚ₊₁ mit p ≤ 4, wobei p ist eine ganze Zahl; CF₂CₚH₂ₚ₊₁ mit p ≤ 4, wobei p eine ganze Zahl ist; CF₂CF₂CₚH₂ₚ₊₁ mit p ≤ 4, wobei p eine ganze Zahl ist; CₘF₂ₘ₊₁ mit m ≤ 4, wobei m eine ganze Zahl ungleich Null ist; und ein Arylrest der Formel (b) : 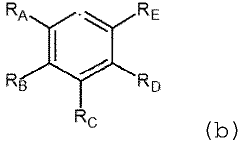
worin R_{A}, R_{B}, R_{C}, R_{D} und R_{E}, die gleich oder verschieden sind, wie zuvor in Formel (B) definiert sind; und,
- mindestens eine zweite kationenextrahierende und hydrophobe organische Verbindung, die aus Kronenether, Kryptanden oder funktionalisierten Calixarenen ausgewählt ist und eine Komplexbildungskonstante der genannten kationischen Spezies aufweist, deren Log K-Wert in Methanol bei 25 °C größer als 3 und kleiner als 9 ist;
b) die Trennung einerseits des vollständig oder teilweise entsalzten und/oder entionisierten flüssigen behandelten Wassers und andererseits der flüssigen organischen Phase, die mit den ionischen Spezies beladen ist; und
c) Mischen bei einer zweiten höheren Temperatur in flüssiger Phase in einem zweiten Reaktor der flüssigen organischen Phase, die mit den ionischen Spezies beladen ist, und flüssigem Regenerationswasser, um anschließend eine regenerierte flüssige organische Phase und flüssiges Regenerationswasser, das mit den ionischen Spezies beladen ist, zu erhalten, wobei der Unterschied zwischen der ersten und der zweiten Temperatur 30 °C bis 150 °C beträgt und die zweite Temperatur höher als die erste Temperatur ist.

2. - Verfahren nach Anspruch 1, **dadurch gekennzeichnet, dass** das Verfahren keinen Schritt umfasst, bei dem der pH-Wert des flüssigen Regenerationswassers signifikant verändert wird, d.h. über eine pH-Schwankung von +/- 2, z.B. +/- 1, in Bezug auf das zu behandelnde Wasser hinaus.

3. - Verfahren nach einem der Ansprüche 1 oder 2, **dadurch gekennzeichnet, dass** das Verfahren die nachfolgenden Schritte umfasst: :
d) Trennen der regenerierten flüssigen organischen Phase und des flüssigen Regenerationswassers, das mit den ionischen Spezies beladen ist ;
e) indirektes thermisches Inkontaktbringen der mit den ionischen Spezies beladenen flüssigen organischen Phase und der regenerierten flüssigen organischen Phase.

4. - Verfahren nach einem der Ansprüche 1 bis 3, **dadurch gekennzeichnet, dass** das Verfahren einen Schritt des Erhitzens von flüssigem Regenerationswasser umfasst, der vor Schritt c) durchgeführt wird.

5. - Verfahren nach einem der Ansprüche 1 bis 4, **dadurch gekennzeichnet, dass** die anionische Spezies Chlorid oder Sulfat ist.

6. - Verfahren nach einem der Ansprüche 1 bis 5, **dadurch gekennzeichnet, dass** die Verbindung (B) eine Verbindung ist, in der X für :

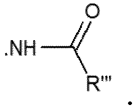

7. - Verfahren nach Anspruch 6, **dadurch gekennzeichnet, daß** die Verbindung (B) durch die Formel dargestellt wird: 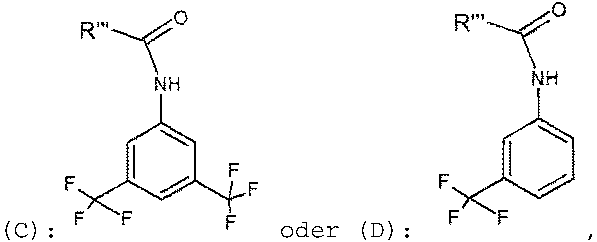
worin R‴ aus den folgenden Radikalen ausgewählt ist: CₘH₂ₘ₊₁ mit m ≤ 20, vorzugsweise ≤ 15, wobei m eine ganze Zahl ist; CₘH₂ₘ₋₁ mit m ≤ 20, wobei m eine ganze Zahl ungleich Null ist; CₘHₙFₚCl_{q}Brₛ mit m ≤ 10, wobei n, p, q, s ganze Zahlen sind, von denen mindestens p, q oder s ungleich Null ist; und ein Arylrest der Formel (b) : 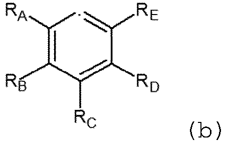
worin mindestens einer der Reste R_{A}, R_{B}, R_{C}, R_{D} und R_{E}, die gleich oder verschieden sind, ein Halogenatom oder eine elektronenanziehende Gruppe, insbesondere einen halogenierten Rest, aus der folgenden Gruppe darstellt: F, Cl, Br; CₘF₂ₘ₊₁ mit m ≤ 4, wobei m eine ganze Zahl ungleich Null ist; CF₂CF₂CₚH₂ₚ₊₁ mit p ≤ 4, wobei p eine ganze Zahl ist; CF₂CₚH₂ₚ₊₁ mit p ≤ 4, wobei p eine ganze Zahl ist ; CH₂CₚF₂ₚ₊₁ mit p ≤ 4, wobei p eine ganze Zahl ist; OCH₂CF₃; C(=O)CF₃; CₘHₙFₚCl_{q}Brₛ mit m ≤ 4, wobei n, p, q, s ganze Zahlen sind, von denen mindestens p, q oder s ungleich Null ist; C(=O)OCₘH₂ₘ₊₁ mit m ≤ 4, wobei m eine ganze Zahl ist; und C(=O)CₘH₂ₘ₊₁ mit m ≤ 4, wobei m eine ganze Zahl ist, wobei der oder die verbleibenden Reste R_{A}, R_{B}, R_{C}, R_{D} und R_{E} gleich oder verschieden aus den folgenden nichtelektronenanziehenden Resten ausgewählt sind: H; CH₃; CH₂CH₃; CH₂CH₂CₚF₂ₚ₊₁ mit p ≤ 4, wobei p ist eine ganze Zahl; CₘH₂ₘ₋₁ mit m ≤ 10, wobei m eine ganze Zahl ungleich Null ist; und CₘH₂ₘ₊₁ mit m ≤ 10, wobei m eine ganze Zahl ungleich Null ist,
wobei nur einer der Reste R_{A} bis R_{E} einer der beiden letztgenannten Reste CₘH₂ₘ₋₁ und CₘH₂ₘ₊₁ sein kann.

8. - Verfahren nach Anspruch 7, **dadurch gekennzeichnet, dass** der Rest R''' n-C₇H₁₅, n-C₉H₁₉, n-C₁₁H₂₃ oder n-C₁₃H₂₇ ist.

9. - Verfahren nach einem der Ansprüche 1 bis 8, **dadurch gekennzeichnet, dass** die zweite organische Verbindung ein Kronenether ist, insbesondere ein Kronenether mit 14 bis 80 Kohlenstoffatomen, und ausgewählt werden kann aus der Gruppe bestehend aus 6,7,9,10,12,13,20,21,23,24-Decahydrodibenzo[b,k] [1,4,7,10,12,16,19] heptaoxacyclohenicosin (DB21C7), Benzo[b] -1,4,7,10,13-pentaoxacyclopentadecan (B15C5), Perhydrobenzo[b]-1,4,7,10,13-pentaoxacyclopentadecan (C15C5), Dicyclohexano-1,4,7,10,13,16-hexaoxacyclooctadecan (DC18C6), Dibenzo[b,k]-1,4,7,10,13,16-hexaoxacyclooctadecan (DB18C6) und 6,7,9,10,12,13,20,21,23,24,26,27-Dodecahydrodibenzo[b,n] [1,4,7,10,13,16,19,22]octaoxacyclotetracosin (DB24C8).

10. - Verfahren nach einem der Ansprüche 1 bis 8, **dadurch gekennzeichnet, dass** die zweite organische Verbindung ein substituiertes Calixaren ist, das beispielsweise 32 bis 80 Kohlenstoffatome, beispielsweise 50 bis 70 Kohlenstoffatome, umfassen kann, wie 4-tert-Butylcalix[4]-aren-O',O',O",O‴-tetraessigsäuretetraethylester, wie Calix[4]Est.

11. - Verfahren nach einem der Ansprüche 1 bis 10, **dadurch gekennzeichnet, dass** die in Schritt a) verwendete flüssige hydrophobe organische Phase auch ein Verflüssigungsmittel enthält.

12. - Verfahren nach Anspruch 11, **dadurch gekennzeichnet, dass** das Verflüssigungsmittel aus der Gruppe ausgewählt ist, die aus polaren aromatischen Lösungsmitteln besteht.

13. - Zusammensetzung zur Durchführung des Verfahrens nach einem der Ansprüche 1 bis 12, **dadurch gekennzeichnet, dass** sie umfasst:
- mindestens eine erste anionensolvatisierende, protische und hydrophobe organische Verbindung, deren pKa in Wasser bei 25°C mindestens 9, vorzugsweise 10,5 beträgt und vorzugsweise kleiner als der pKa von Wasser bei 25°C oder zumindest kleiner als 15 bei 25°C ist und deren Löslichkeit in Wasser bei 25°C weniger als 0,01 mol/l beträgt, wobei die erste Verbindung eine Verbindung ist, die durch die Formel (B) dargestellt wird: 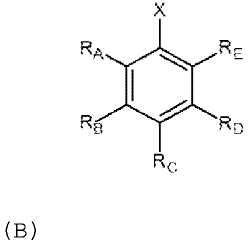
in der mindestens einer der Reste R_{A}, R_{B}, R_{C}, R_{D} und R_{E}, die gleich oder verschieden sind, ein Halogenatom oder eine elektronenanziehende Gruppe aus der folgenden Gruppe ist: F, Cl, Br; CₘF₂ₘ₊₁ mit m ≤ 4, wobei m eine ganze Zahl ungleich Null ist; CF₂CF₂CₚH₂ₚ₊₁ mit p ≤ 4, wobei p eine ganze Zahl ist; CF₂CₚH₂ₚ₊₁ mit p ≤ 4, wobei p eine ganze Zahl ist; CH₂CₚF₂ₚ₊₁ mit p ≤ 4, wobei p eine ganze Zahl ist; OCH₂CF₃; C(=O)CF₃; CₘHₙFₚCl_{q}Brₛ mit m ≤ 4, wobei n, p, q, s ganze Zahlen sind, von denen mindestens p, q oder s nicht Null ist; C(=O)OCₘH₂ₘ₊₁ mit m ≤ 4, wobei m eine ganze Zahl ist; und C(=O)CₘH₂ₘ₊₁ mit m ≤ 4, wobei m eine ganze Zahl ist,
der oder die verbleibenden Reste R_{A}, R_{B}, R_{C}, R_{D} und R_{E} gleich oder verschieden aus den folgenden nichtelektronenanziehenden Resten ausgewählt sind: H; CH₃; CH₂CH₃; CH₂CH₂CₚF₂ₚ₊₁ mit p ≤ 4, wobei p ist eine ganze Zahl; CₘH₂ₘ₋₁ mit m ≤ 10, wobei m eine ganze Zahl ungleich Null ist; und CₘH₂ₘ₊₁ mit m ≤ 10, wobei m eine ganze Zahl ungleich Null ist; wobei nur einer der Reste R_{A} bis R_{E} einer der beiden letztgenannten Reste CₘH₂ₘ₋₁ und CₘH₂ₘ₊₁ sein kann ;
und worin X aus den folgenden Radikalen ausgewählt ist: OH; 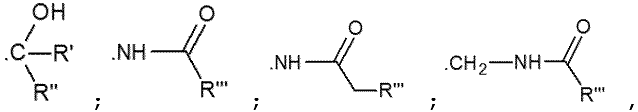
wobei R' und R", die gleich oder verschieden sind, aus den folgenden Resten ausgewählt sind: CₙH₂ₙ₋₁ mit n ≤ 4, wobei n eine ganze Zahl ungleich Null ist; CₙH₂ₙ₊₁ mit n ≤ 4, wobei n eine ganze Zahl ungleich Null ist; CH₂CH₂CₚF₂ₚ₊₁ mit p ≤ 4, wobei p ist eine ganze Zahl; CH₂CₚF₂ₚ₊₁ mit p ≤ 4, wobei p ist eine ganze Zahl; CF₂CₚH₂ₚ₊₁ mit p ≤ 4, wobei p eine ganze Zahl ist ; CF₂CF₂CₚH₂ₚ₊₁ mit p ≤ 4, wobei p eine ganze Zahl ist; CₘF₂ₘ₊₁ mit m ≤ 4, wobei m eine ganze Zahl ungleich Null ist; CₘHₙFₚCl_{q}Brₛ mit m ≤ 4, wobei n, p, q, s ganze Zahlen sind, von denen mindestens p, q oder s ungleich Null ist; und ein Arylradikal der Formel (b) : 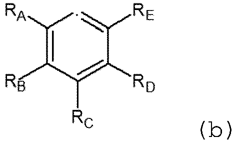
worin R_{A}, R_{C}, R_{C}, R_{D} und R_{E}, die gleich oder verschieden sind, wie zuvor in Formel (B) definiert sind;
und wobei R‴ aus den folgenden Radikalen ausgewählt ist: CₘH₂ₘ₊₁ mit m ≤ 20, wobei m eine ganze Zahl ist; C₍ₘ₎H₂ₘ₋₁ mit m ≤ 20, wobei m eine ganze Zahl ungleich Null ist; CₘHₙFₚCl_{q}Brₛ mit m ≤ 10, wobei n, p, q, s ganze Zahlen sind, von denen mindestens p, q oder s ungleich Null ist; CH₂CH₂CₚF₂ₚ₊₁ mit p ≤ 4, wobei p eine ganze Zahl ist; CH₂CₚF₂ₚ₊₁ mit p ≤ 4, wobei p ist eine ganze Zahl; CF₂CₚH₂ₚ₊₁ mit p ≤ 4, wobei p eine ganze Zahl ist; CF₂CF₂CₚH₂ₚ₊₁ mit p ≤ 4, wobei p eine ganze Zahl ist; CₘF₂ₘ₊₁ mit m ≤ 4, wobei m eine ganze Zahl ungleich Null ist; und ein Arylrest der Formel (b) : 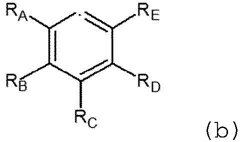
- worin R_{A}, R_{C}, R_{C}, R_{D} und R_{E}, die gleich oder verschieden sind, wie zuvor in Formel (B) definiert sind;
- mindestens eine zweite kation extrahierende hydrophobe organische Verbindung, die aus Kronenether, Kryptanden oder funktionalisierten Calixarenen ausgewählt ist und eine Komplexbildungskonstante der genannten kationischen Spezies aufweist, deren Log K-Wert in Methanol bei 25 °C größer als 3 und kleiner als 9 ist.

## Claims

1. - Method for treating saline water by thermal deionisation comprising the extraction of at least two ionic species, said ionic species comprising an anionic species and a cationic species and being present in the water to be treated, said method comprising the following steps:
a) mixing in a first reactor, at a first temperature, between an hydrophobic organic liquid phase and the saline water to be treated, said water to be treated being in the liquid state, in order to subsequently obtain a treated liquid water desalted and/or deionized in whole or in part and an hydrophobic organic liquid phase charged with said ionic species, said hydrophobic phase comprising:
- at least a first protic, hydrophobic anion solvating organic compound, whose pKa in water at 25°C is at least 9, preferably 10.5 and is preferably less than the pKa of water at 25°C, or at least less than 15 at 25°C and whose solubility in water at 25°C is at least less than 0.1 mol/liter, said first compound being a compound of Formula (B) : 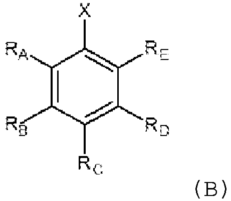
in which at least any one of the radicals R_{A}, R_{B}, R_{C}, R_{D} and R_{E}, which are identical or different, is a halogen atom or an electron-withdrawing group, of the following group: F, Cl, Br; CₘF₂ₘ₊₁ with m ≤ 4, where m is a non-zero integer; CF₂CF₂CₚH₂ₚ₊₁ with p ≤ 4, where p is an integer; CF₂CₚH₂ₚ₊₁ with p ≤ 4, where p is an integer; CH₂CₚF₂ₚ₊₁ with p ≤ 4, where p is an integer; OCH₂CF₃; C(=O)CF₃; CₘHₙFₚCl_{q}Brₛ with m ≤ 4, where n, p, q, s are integers of which at least p, q or s is non-zero; C(=O)OCₘH₂ₘ₊₁ with m ≤ 4, where m is an integer; and C(=O)CₘH₂ₘ₊₁ with m ≤ 4, where m is an integer,
the remaining radical(s) R_{A}, R_{B}, R_{C}, R_{D} and R_{E} are chosen, identical or different, from the following non-electron-withdrawing radicals: H; CH₃; CH₂CH₃; CH₂CH₂CₚF₂ₚ₊₁ with p ≤ 4, where p is an integer; CₘH₂ₘ₋₁ with m ≤ 10, where m is a non-zero integer; and CₘH₂ₘ₊₁ with m ≤ 10, where m is a non-zero integer; where only one of the radicals R_{A} to R_{E} can be one of these last two radicals CₘH₂ₘ₋₁ and CₘH₂ₘ₊₁;
and wherein X is selected from the following radicals: OH; 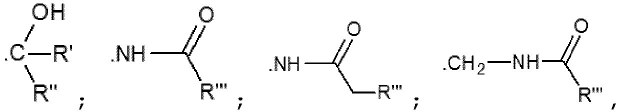
where R' and R'', identical or different, are chosen from the following radicals: CₙH₂ₙ₋₁ with n ≤ 4, where n is a non-zero integer; CₙH₂ₙ₊₁ with n ≤ 4, where n is a non-zero integer; CH₂CH₂CₚF₂ₚ₊₁ with p ≤ 4, where p is an integer; CH₂CₚF₂ₚ₊₁ with p ≤ 4, where p is an integer; CF₂CₚH₂ₚ₊₁ with p ≤ 4, where p is an integer; CF₂CF₂CₚH₂ₚ₊₁ with p ≤ 4, where p is an integer; CₘF₂ₘ₊₁ with m ≤ 4, where m is a non-zero integer; CₘHₙFₚCl_{q}Brₛ with m ≤ 4, where n, p, q, s are integers of which at least p, q or s is non-zero; and an aryl radical of formula (b): 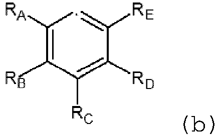
where R_{A}, R_{B}, R_{C}, R_{D} and R_{E}, identical or different, are as defined above in formula (B);
and wherein R''' is selected from the following radicals: CₘH₂ₘ₊₁ with m ≤ 20, where m is an integer; CₘH₂ₘ₋₁ with m ≤ 20, where m is a non-zero integer; CₘHₙFₚC_{lq}Brₛ with m ≤ 10, where n, p, q, s are integers of which at least p, q or s is non-zero; CH₂CH₂CₚF₂ₚ₊₁ with p ≤ 4, where p is an integer; CH₂CₚF₂ₚ₊₁ with p ≤ 4, where p is an integer; CF₂CₚH₂ₚ₊₁ with p ≤ 4, where p is an integer; CF₂CF₂CₚH₂ₚ₊₁ with p ≤ 4, where p is an integer; CₘF₂ₘ₊₁ with m ≤ 4, where m is a non-zero integer; and an aryl radical of formula (b): 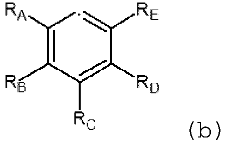
where R_{A}, R_{B}, R_{C}, R_{D} and R_{E}, which may be identical or different, are as defined above in formula (B); and
- at least a second hydrophobic, cation extracting organic compound selected from Ether-crowns, Cryptands or functionalized Calixarenes and having a complexing constant of said cationic species whose log K value, in methanol at 25°C, is greater than 3 and less than 9;
b) separating, on one hand, of said treated liquid water desalted and/or deionized in whole or in part and on the other hand of said organic liquid phase charged with said ionic species ; and
c) mixing, at a second, higher temperature, in the liquid phase, in a second reactor, of said organic liquid phase, charged with ionic species, with regeneration liquid water, for the subsequent production of a regenerated organic liquid phase and of a regeneration liquid water charged with ionic species, the difference between said first and second temperatures going from 30°C to 150°C, the second temperature being higher than the first temperature.

2. - Method according to claim 1, **characterized by** the fact that the method does not include a step in which the pH of the regeneration liquid water is significantly modified, that is to say, beyond a variation of pH of +/-2, for example ± 1 relative to the water to be treated.

3. - Method according to one of claims 1 and 2, **characterized by** the fact that said method comprises the following subsequent steps:
d) separating said regenerated organic liquid phase and regeneration liquid water charged with said ionic species ;
e) indirect thermal contacting of said organic liquid phase charged with ionic species and of said regenerated organic liquid phase.

4. - Method according to any one of claims 1 to 3, **characterized by** the fact that the method comprises a step of heating the regeneration liquid water carried out before step c).

5. - Method according to any one of claims 1 to 3, **characterized by** the fact that the anionic species is chloride or sulphate.

6. - Method according to one of claims 1 to 5, **characterized by** the fact that compound (B) is a compound in which X represents:

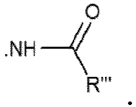

7. - Method according to claim 6, **characterized by** the fact that compound (B) is represented by formula: 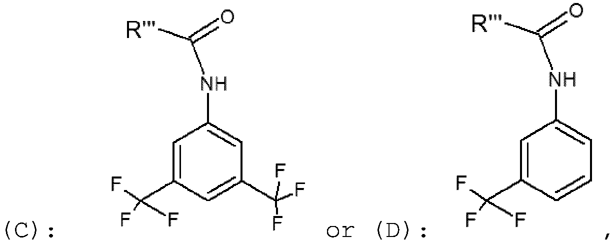
in which R‴ is chosen from the following radicals: CₘH₂ₘ₊₁ with m ≤ 20, preferably ≤ 15 where m is an integer; CₘH₂ₘ₋₁ with m ≤ 20, where m is a non-zero integer; CₘHₙFₚCl_{q}Brₛ with m ≤ 10, where n, p, q, s are integers of which at least p, q or s is non-zero; and an aryl radical of formula (b): 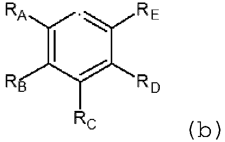
in which at least one of the radicals R_{A}, R_{B}, R_{C}, R_{D} and R_{E}, identical or different, is an halogen atom or an electron-withdrawing group, in particular a halogenated radical, of the following group: F, Cl, Br; CₘF₂ₘ₊₁ with m ≤ 4, where m is a non-zero integer; CF₂CF₂CₚH₂ₚ₊₁ with p ≤ 4, where p is an integer; CF₂CₚH₂ₚ₊₁ with p ≤ 4, where p is an integer: CH₂CₚF₂ₚ₊₁ with p ≤ 4, where p is an integer; OCH₂CF₃ ; C(=O)CF₃; CₘHₙFₚCl_{q}Brₛ with m ≤ 4, where n, p, q, s are integers of which at least p, q or s is non-zero; C(=O)OCₘH₂ₘ₊₁ with m ≤ 4, where m is an integer; and C(=O)CₘH₂ₘ₊₁ with m ≤ 4, where m is an integer, the remaining radical(s) R_{A}, R_{B}, R_{C}, R_{D} and R_{E} are chosen, identical or different, from the following non-electron withdrawing radicals: H; CH₃; CH₂CH₃; CH₂CH₂CₚF_{2p + 1} with p ≤ 4, where p is an integer; CₘH₂ₘ₋₁ with m ≤ 10, where m is a non-zero integer; and CₘH₂ₘ₊₁ with m ≤ 10, where m is a non-zero integer,
where only one of the radicals R_{A} to R_{E} can be one of these last two radicals CₘH₂ₘ₋₁ and CₘH₂ₘ₊₁.

8. - Method according to claim 7, **characterized by** the fact that radical R‴ is n-C₇H₁₅, n-C₉H₁₉, n-C₁₁H_{z3} or n-C₁₃H₂₇.

9. - Method according to one of claims 1 to 8, **characterized by** the fact that the second hydrophobic organic compound is a crown ether having from 14 to 80 carbon atoms, and can be chosen from the group consisting of 6,7,9,10,12,13,20,21,23,24-decahydrodibenzo[b,k] [1,4,7,10,12,16,19] heptaoxa-cyclohenicosine (DB21C7), benzo[b]-1,4,7,10,13-pentaoxacyclopentadecane (B15C5), perhydrobenzo[b]-1,4,7,10,13-pentaoxacyclopentadecane (C15C5), dicyclohexano-1,4,7,10,13,16-hexaoxacyclooctadecane (DC18C6), dibenzo[b,k]-1,4,7,10,13,16-hexaoxacyclooctadecane (DB18C6) and 6,7,9,10,12,13,20,21,23,24,26,27-dodécahydrodibenzo[b,n] [1,4,7,10,13,16,19,22]octaoxa-cyclotetracosine (DB24C8).

10. - Method according to one of claims 1 to 8, **characterized by** the fact that the second organic compound is a substituted calixarene which can comprise, for example, from 32 to 80 carbon atoms, for example from 50 to 70 carbon atomes, such as 4-tert-butylcalix[4]-arene-O,O',O",O‴-tetraacetic acid tetraethyl ester, such as Calix[4]Est.

11. - Method according to one of claims 1 to 10, **characterized by** the fact that the hydrophobic organic liquid phase of step a) also comprises a fluidifying agent.

12. - Method according to claim 11, **characterized by** the fact that the fluidifying agent is selected from the group consisting of polar aromatic solvents.

13. - Composition for carrying out the method according to one of claims 1 to 12, **characterized by** the fact that it comprises:
- at least a first protic, hydrophobic anion solvating organic compound, whose pKa in water at 25°C is at least 9, preferably 10.5 and is preferably less than the pKa of water at 25°C, or at least less than 15 at 25°C and whose solubility in water at 25°C is less than 0.1 mol/liter, said first compound being a compound of Formula (B): 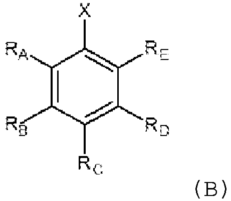
in which at least any one of the radicals R_{A}, R_{B}, R_{C}, R_{D} and R_{E}, which are identical or different, is a halogen atom or an electron-withdrawing group, of the following group: F, Cl, Br; CₘF₂ₘ₊₁ with m ≤ 4, where m is a non-zero integer; CF₂CF₂CₚH₂ₚ₊₁ with p ≤ 4, where p is an integer; CF₂CₚH₂ₚ₊₁ with p ≤ 4, where p is an integer; CH₂CₚF₂ₚ₊₁ with p ≤ 4, where p is an integer; OCH₂CF₃; C(=O)CF₃; CₘHₙFₚCl_{q}Brₛ with m ≤ 4, where n, p, q, s are integers of which at least p, q or s is non-zero; C(=O)OCₘH₂ₘ₊₁ with m ≤ 4, where m is an integer; and C(=O)CₘH₂ₘ₊₁ with m ≤ 4, where m is an integer,
the remaining radical(s) R_{A}, R_{B}, R_{C}, R_{D} and R_{E} are chosen, identical or different, from the following non-electron withdrawing radicals: H; CH₃; CH₂CH₃; CH₂CH₂CₚF₂ₚ₊₁ with p ≤ 4, where p is an integer; CₘH₂ₘ₋₁ with m ≤ 10, where m is a non-zero integer; and CₘH₂ₘ₊₁ with m ≤ 10, where m is a non-zero integer; where only one of the radicals R_{A} to R_{E} may be one of these last two radicals CₘH₂ₘ₋₁ and CₘH₂ₘ₊₁;
and wherein X is selected from the following radicals: OH; 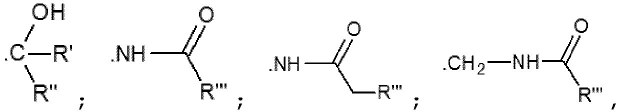
where R' and R'', which may be identical or different, are chosen from the following radicals: CₙH₂ₙ₋₁ with n ≤ 4, where n is a non-zero integer; CₙH₂ₙ₊₁ with n ≤ 4, where n is a non-zero integer; CH₂CH₂CₚF₂ₚ₊₁ with p ≤ 4, where p is an integer; CH₂CₚF₂ₚ₊₁ with p ≤ 4, where p is an integer; CF₂CₚH₂ₚ₊₁ with p ≤ 4, where p is an integer; CF₂CF₂CₚH₂ₚ₊₁ with p ≤ 4, where p is an integer; CₘF₂ₘ₊₁ with m ≤ 4, where m is a non-zero integer; CₘHₙFₚCl_{q}Brₛ with m ≤ 4, where n, p, q, s are integers of which at least p, q or s is non-zero; and an aryl radical of formula (b): 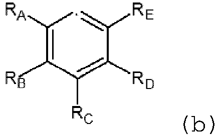
where R_{A}, R_{B}, R_{C}, R_{D} and R_{E}, which may be identical or different, are as defined above in formula (B);
and wherein R''' is selected from the following radicals: CₘH₂ₘ₊₁ with m ≤ 20, where m is an integer; CₘH₂ₘ₋₁ with m ≤ 20, where m is a non-zero integer; CₘHₙFₚC_{lq}Brₛ with m ≤ 10, where n, p, q, s are integers of which at least p, q or s is non-zero; CH₂CH₂CₚF₂ₚ₊₁ with p ≤ 4, where p is an integer; CH₂CₚF₂ₚ₊₁ with p ≤ 4, where p is an integer; CF₂CₚH₂ₚ₊₁ with p ≤ 4, where p is an integer; CF₂CF₂CₚH₂ₚ₊₁ with p ≤ 4, where p is an integer; CₘF₂ₘ₊₁ with m ≤ 4, where m is a non-zero integer; and an aryl radical of formula (b): 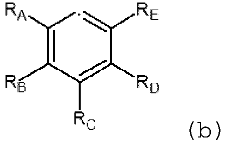
- where R_{A}, R_{B}, R_{C}, R_{D} and R_{E}, which may be identical or different, are as defined above in formula (B); and
- at least a second hydrophobic, cation extracting organic compound selected from Ether-crowns, Cryptands or functionalized Calixarenes and having a complexing constant of said cationic species whose log K value, in methanol at 25°C, is greater than 3 and less than 9.
